# EUROPEAN PATENT SPECIFICATION

(11) **EP 4 637 472 B1**
(45) Date of publication and mention of the grant of the patent: **14.10.2026**
(21) Application number: 23837318.7
(22) Date of filing: 21.12.2023
(51) Int. Cl.: A45D 19/00, A45D 19/02, A61K 8/40, A61K 8/84, A61Q 5/06

(54) **PROCESS FOR DYEING KERATIN HAIR FIBERS WHICH INVOLVES SPRAYING THE KERATIN HAIR FIBERS WITH A COMPOSITION COMPRISING A (POLY)CARBODIIMIDE COMPOUND AND A COLOURING AGENT USING AN AIRBRUSH**
VERFAHREN ZUM FÄRBEN VON KERATIN-HAARFASERN, BEI DEM DIE KERATIN-HAARFASERN MIT EINER ZUSAMMENSETZUNG, BESTEHEND AUS EINER (POLY)CARBODIIMID-VERBINDUNG UND EINEM FARBSTOFF, MIT EINER AIRBRUSH BESPRÜHT WERDEN
PROCÉDÉ DE TEINTURE DES FIBRES CAPILLAIRES KÉRATINIQUES CONSISTANT À PULVÉRISER SUR LES FIBRES CAPILLAIRES KÉRATINIQUES UNE COMPOSITION COMPRENANT UN COMPOSÉ (POLY)CARBODIIMIDE ET UN COLORANT À L'AIDE D'UN AÉROGRAPHE

(30) Priority: 21.12.2022 FR 2214066
(43) Date of publication of application: 29.10.2025
(73) Proprietor: L'OREAL, 75008 Paris (FR)
(72) Inventor: GRACIA, Marie, 93400 SAINT OUEN (FR); POURILLE, Chrystel, 93400 SAINT OUEN (FR); BROTHIER, Fabien, 93400 SAINT OUEN (FR)
(74) Representative: Casalonga
(86) International application number: PCT/EP2023/087312
(87) International publication number: WO 2024/133719

(56) References cited:
- WO-A1-2013/045379
- WO-A1-2021/099515
- WO-A1-2021/099517
- WO-A1-2022/189570
- WO-A1-2022/189573
- WO-A1-2022/189575
- WO-A1-2022/189576
- FR-A1- 3 003 752

## Description

The present invention relates to a process for dyeing keratin hair fibers which involves spraying the keratin hair fibers with a composition C comprising at least one (poly)carbodiimide compound and a colouring agent using an airbrush.

### Technical field

In the field of dyeing keratin hair fibers, in particular human keratin fibres, it is already known practice to dye keratin hair fibers via various techniques using direct dyes or pigments for non-permanent colouring, or dye precursors for permanent colouring.

There are essentially three types of process for dyeing the keratin hair fibers:
a) "permanent" dyeing, the function of which is to afford a substantial modification to the natural colour and which uses oxidation dyes which penetrate into the keratin hair fibers and forms the dye via an oxidative condensation process;
b) non-permanent, semi-permanent or direct colouring, which does not use the oxidative condensation process and withstands four or five shampoo washes; it consists in colouring keratin fibres with colour compositions containing direct dyes;
c) temporary dyeing, which gives rise to a modification of the natural colour of the keratin hair fibers that remains from one shampoo wash to the next, and which serves to enhance or correct a shade that has already been obtained. It may also be likened to a "makeup" process.

Another dyeing method consists in using pigments. Specifically, the use of pigment on the surface of keratin fibres generally makes it possible to obtain colourings that are visible on dark keratin hair fibers, since the surface pigment masks the natural colour of the fibre. However, the colourings obtained via this dyeing method have the drawback of having poor resistance to shampoo washing and also to external agents such as sebum, perspiration, brushing and/or rubbing.

There is thus still a need for a process for dyeing keratin hair fibers which has the advantage of obtaining a uniform coloured coating on the keratin hair fibers, while at the same time forming a coating that is persistent with respect to shampoo washing and to the various stresses to which the keratin hair fibers may be subjected such as brushing and/or rubbing, without degradation of the cosmetic properties of the keratin hair fibers. Documents WO 2022/189575, WO 2022/189573,WO 2021/099517, WO 2022/189570, WO 2021/099515, and WO 2022/189576 disclose compositions for dyeing hair comprising at least one (poly)carbodiimide compound.

Thus, the aim of the present invention is to develop a process for dyeing keratin hair fibers which has the advantage of obtaining a smooth and uniform coloured coating on the keratin hair fibers, while at the same time forming a coating that is persistent with respect to shampoo washing and to the various stresses to which the keratin hair fibers may be subjected, such as brushing and/or rubbing, without degradation of the cosmetic properties of the keratin hair fibers.

### Disclosure of the invention

One subject of the present invention is thus a process for dyeing keratin hair fibers which involves spraying the keratin hair fibers with at least one composition C comprising:
- at least one (poly)carbodiimide compound; and
- at least one colouring agent chosen from pigments, direct dyes and mixtures thereof, using an airbrush.

By virtue of the process for dyeing keratin hair fibers according to the invention, coloured coatings are obtained on the keratin hair fibers that make it possible to obtain a colouring that is visible on all keratin hair fibers types and persistent with respect to shampoo washing, while at the same time preserving the physical qualities of the keratin hair fibers. Such a coating may be resistant to the external stresses to which the keratin hair fibers may be subjected, such as blow-drying and perspiration. In particular, it allows a thin, uniform deposit to be obtained easily, without any running, and which dries quickly.

Unlike a conventional aerosol device (pressurized packaging of the composition to be sprayed), the composition can be stored in non-pressurized form (i.e. at atmospheric pressure) in a container carried by or connected to the airbrush prior to use.

Another subject of the invention is a spraying system comprising an airbrush, and at least one compartment containing said composition C.

The invention may be understood more clearly on reading the detailed description of the airbrush that follows, of non-limiting examples of use thereof, and on inspection of the attached drawings, in which:
[Fig 1] represents, schematically and partially, the airbrush and an example of a compressed air feed system for the airbrush,
[Fig 2] represents a perspective view of the airbrush.

For the purposes of the present invention, the term *"colouring that is persistent with respect to shampoo washing"* means that the colouring obtained persists after one shampoo wash, preferably after three shampoo washes.

The term "*at least one"* means one or more.

Unless otherwise indicated, the limits of a range of values are included in that range, notably in the expressions "between" and "ranging from ... to ...".

The invention is not limited to the examples illustrated. The characteristics of the various examples may notably be combined within variants which are not illustrated.

For the purposes of the present invention and unless otherwise indicated:
- an "*alkyl*" radical denotes a linear or branched saturated radical containing, for example, from 1 to 20 carbon atoms;
- an "*aminoalkyl*" radical denotes an alkyl radical as defined previously, said alkyl radical comprising an NH₂ group;
- *a* "*hydroxyalkyl*" radical denotes an alkyl radical as defined previously, said alkyl radical comprising an OH group;
- an "*alkylene*" radical denotes a linear or branched divalent saturated C₂-C₄ hydrocarbon-based group such as methylene, ethylene or propylene;
- a "*cycloalkyl*" or "*alicycloalkyl*" radical denotes a cyclic saturated monocyclic or bicyclic, preferably monocyclic, hydrocarbon-based group comprising from 1 to 3 rings, preferably 2 rings, and comprising from 3 to 24 carbon atoms, in particular comprising from 3 to 20 carbon atoms, more particularly from 3 to 13 carbon atoms, even more particularly from 3 to 12 carbon atoms, preferably between 5 and 10 carbon atoms, such as cyclopropyl, cyclopentyl, cyclohexyl, cycloheptyl or norbornyl, in particular cyclopropyl, cyclopentyl or cyclohexyl, it being understood that the cycloalkyl radical may be substituted with one or more (C₁-C₄)alkyl groups such as methyl; preferably, the cycloalkyl radical is then an isobornyl group;
- a "*cycloalkylene*" radical denotes a divalent cycloalkyl group with "*cycloalkyl*" as defined previously, preferably of C₃-C₁₂;
- an "*aryl*" radical is a monocyclic, bicyclic or tricyclic, fused or non-fused, unsaturated and aromatic hydrocarbon-based cyclic radical, comprising from 6 to 14 carbon atoms, preferably between 6 and 12 carbon atoms; preferably, the aryl group comprises 1 ring of 6 carbon atoms such as phenyl, naphthyl, anthryl, phenanthryl and biphenyl, it being understood that the aryl radical may be substituted with one or more (C₁-C₄)alkyl groups such as methyl, preferably tolyl, xylyl, or methylnaphthyl; preferably, the aryl group represents phenyl;
- an "*arylene*" radical is a divalent aryl radical with "*aryl*" as defined previously; preferably, arylene represents phenylene;
- a "*heterocyclic*" radical denotes a saturated or unsaturated, non-aromatic or aromatic, monocyclic or polycyclic hydrocarbon-based radical, comprising one or more heteroatoms, preferably from 1 to 5 atoms chosen from O, S or N, including from 3 to 20 ring members, preferably between 5 and 10 ring members, such as imidazolyl, pyrrolyl and furanyl;
- a "*heterocycloalkylene*" radical is a divalent heterocyclic group with *"heterocyclic"* as defined previously;
- an *"aryloxy"* radical denotes an aryl-oxy radical with "*aryl*" as defined previously;
- an "*alkoxy*" radical denotes an alkyl-oxy radical with "*alkyl*" as defined previously;
- an *"acyloxy"* radical denotes an ester radical R-C(O)-O- with R being an alkyl group as defined previously;
- *a* "*reactive*" group is a group that is capable of forming a covalent bond with another identical or different group, by chemical reaction.

Unless otherwise indicated, when compounds are mentioned in the present patent application, this also includes the optical isomers thereof, the geometrical isomers thereof, the tautomers thereof or the salts thereof, alone or as a mixture.

The term "hair keratin fibres" means the keratin hair fibers or the hair. In other words, the expressions "hair keratin fibres", "keratin hair fibers" and "hair" are equivalent in the continuation of the description.

For the purposes of the present invention, the term "keratin hair fibers" means head keratin hair fibers. This term does not correspond to bodily hairs, the eyebrows or the eyelashes.

### Polycarbodiimide compound

Composition C used in the context of the process according to the invention comprises at least one (poly)carbodiimide compound.

The composition may comprise at least two different (poly)carbodiimide compounds, present as a mixture in the composition.

The term *"(poly)carbodiimide compound"* means a compound comprising one or more carbodiimide groups, preferably at least two carbodiimide groups, more preferentially at least three carbodiimide groups; in particular, the number of carbodiimide groups does not exceed 200, preferably 150, more preferentially 100.

The term *"carbodiimide group"* means a divalent linear triatomic fraction of general formula -(N=C=N)-.

The (poly)carbodiimide compound(s) according to the invention may optionally comprise in their structure one or more reactive groups different from carbodiimide groups, chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups.

The reactive group(s) other than the carbodiimide groups may be side or end groups. Preferably, the (poly)carbodiimide compound(s) comprise one or more end groups different from carbodiimide groups, preferably one or more end groups chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups.

According to a particular embodiment, the (poly)carbodiimide compound is chosen from the compounds of formula (I) below: 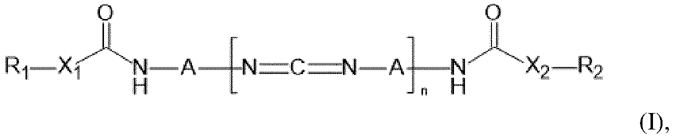 in which:
- X₁ and X₂ independently represent an oxygen atom O, a sulfur atom S or an NH group;
- R₁ and R₂ independently represent a group chosen from a hydrocarbon-based radical, preferably alkyl, optionally interrupted with one or more heteroatoms, a group chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups, and a hydrocarbon-based radical, preferably alkyl, optionally interrupted with one or more heteroatoms and with one or more groups chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups;
- n denotes an integer ranging from 1 to 1000; and
- A is a monomer chosen from the compounds below:

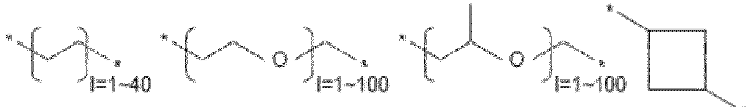

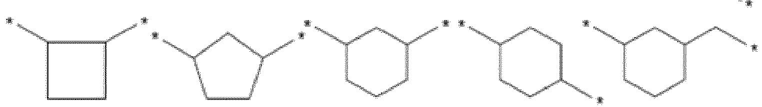

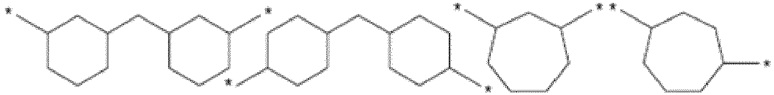

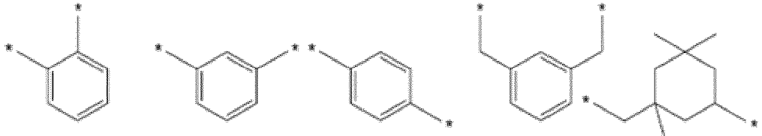

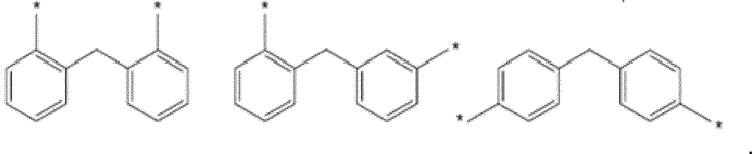

According to another embodiment, the (poly)carbodiimide compound is chosen from the compounds of formula (I') below: 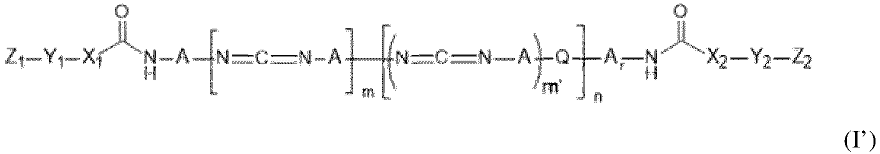 in which:
- X₁ and X₂ independently represent an oxygen atom O, a sulfur atom S or an NH group;
- Y₁ and Y₂ independently represent a divalent organic radical chosen from a saturated C₁ to C₃₆ aliphatic group or a C₆ to C₂₄ aromatic or alkylaromatic group, the aliphatic or aromatic group optionally comprising one or more non-pendent heteroatoms, such as a nitrogen atom, an oxygen atom, a sulfur atom, or combinations thereof;
- Z₁ and Z₂ independently represent a reactive end group or an inert end group;
- as inert end group, Z₁ and Z₂ may represent, independently, a saturated, linear or branched or cyclic C₁ to C₅₀ aliphatic group, or a C₆ to C₁₈ aromatic group, said aliphatic and aromatic groups optionally comprising from 1 to 10 heteroatoms chosen from nitrogen, oxygen, sulfur and combinations thereof, and the aliphatic or aromatic group may be partially or totally fluorinated; in this variant, Z₁ and Z₂ comprise a bonding group CG connecting Z₁ to Y₁ and Z₂ to Y₂, the group CG possibly being a single covalent bond, a saturated C-C bond, an unsaturated covalent C-C bond, an amide group, an ester group, a carbonate group, a thioester group, an ether group, a urethane group, a thiourethane group or a urea group;
- as reactive end group, Z₁ and Z₂ may be chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups;
- Q represents an organopolymer or an organooligomer comprising repeating units of saturated, linear or branched or cyclic aliphatic groups, or of aromatic groups or alkylaromatic groups, coupled via carbonate, ester, ether, amide, urethane or urea repeating bonds or combinations thereof;
- A represents a divalent aliphatic, aromatic, alkylaromatic or linear, saturated, branched or cyclic radical containing from 2 to 30 carbon atoms, which may optionally comprise one or more non-pendent heteroatoms such as a nitrogen atom, an oxygen atom, a sulfur atom, or combinations thereof, in the aliphatic chain or the aromatic chain;
- r denotes an integer equal to 0 or 1;
- m denotes an integer ranging from 0 to 1000, preferably equal to 0 or 1;
- m' denotes an integer ranging from 0 to 1000, preferably equal to 0 or 1;
- n denotes an integer ranging from 0 to 1000, preferably equal to 0 or 1, with m + (m'*n) ≥ 2.

Preferably, Z₁ and Z₂ independently represent a reactive end group; more preferentially, Z₁ and Z₂ independently represent a group chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups.

Such (poly)carbodiimide compounds are sold, for example, by the company Stahl B.V, under the name Permutex XR, or under the name RelcaLink10., under the name Picassian XL and Nisshinbo compounds sold under the name Carbodilite with the series V-02, V-02-L2, SV-02, E-02, V-10, SW-12G, E-03A, E-04DG-T, E-05, V-04, V-02B, V-04PF, V-05.

Preferably, the (poly)carbodiimide compound(s) are chosen from the compounds of formula (II) below: 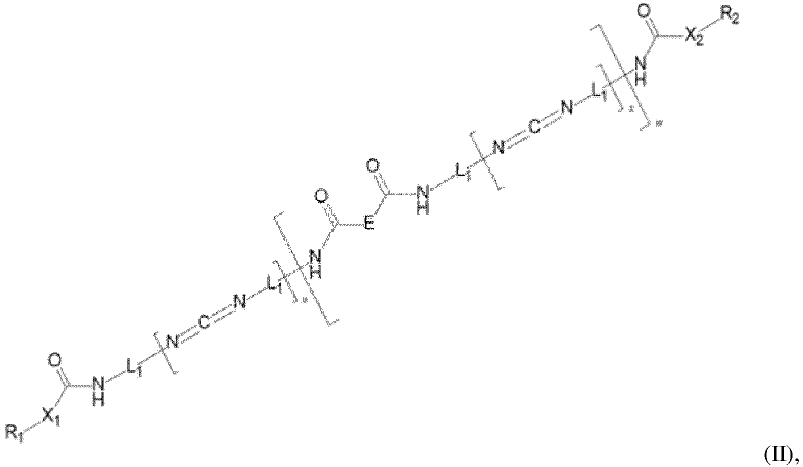 in which:
- X₁ and X₂ independently represent an oxygen atom O, a sulfur atom S or an NH group;
- R₁ and R₂ independently represent a hydrocarbon-based radical optionally interrupted with one or more heteroatoms;
- n and z denote an integer ranging from 1 to 20, with n+z ≥ 2 and w denotes an integer ranging from 1 to 3;
- L₁ independently represents a C₁-C₁₈ divalent aliphatic hydrocarbon-based radical, a C₃-C₁₅ cycloalkylene radical, a C₃-C₁₂ heterocycloalkylene group or a C₆-C₁₄ arylene group, and mixtures thereof;
- E independently represents a group chosen from:

   -O-R₃-O-; -S-R₄-S-; -R₅-N(R₆)-R₄-N(R₆)-R₅-;

   in which R₃ and R₄ independently represent a divalent hydrocarbon-based radical optionally interrupted with one or more heteroatoms;
- R₅ independently represents a covalent bond or a saturated divalent hydrocarbon-based radical, optionally interrupted with one or more heteroatoms;
- R₆ independently represents a hydrogen atom or a hydrocarbon-based radical, optionally interrupted with one or more heteroatoms.

The term *"hydrocarbon-based radical"* means a saturated or unsaturated, linear or branched radical containing from 1 to 300 carbon atoms, preferably from 1 to 250 carbon atoms, more preferentially from 1 to 200 carbon atoms. Preferably, the hydrocarbon-based radical is a saturated linear radical.

The hydrocarbon-based radical may comprise one or more cyclic groups.

The hydrocarbon-based radical may be interrupted with one or more heteroatoms, in particular chosen from O, S or N and/or substituted with one or more cations, anions or zwitterions or cationic groups such as ammonium, anionic groups such as carboxylate, or zwitterionic groups, and/or comprising a metal ion which may be incorporated in the form of a salt.

The term "*heteroatom(s)*" means an oxygen O, sulfur S or nitrogen N atom, and also halogen atoms such as Cl, F, Br and I. If the heteroatom is included in the chain of the hydrocarbon-based radical, the heteroatom is preferably chosen from oxygen O, sulfur S or nitrogen N atoms.

Preferably, X₁ and X₂ independently represent an oxygen atom.

Preferably, R₁ and R₂ are independently chosen from dialkylamino alcohols, alkyl esters of hydroxycarboxylic acid and monoalkyl ethers of (poly)alkylene glycol, in which a hydroxyl group has been removed, and mixtures thereof.

In a preferred embodiment, R₁ and R₂ are independently chosen from groups (i) to (iv) below:
(i) the compound of formula (III) below:

   R₇-O-C(O)-C(R₈)(H)- (III),

   in which R₇ represents a C₁-C₃ alkyl group and R₈ represents a hydrogen atom or a C₁-C₃ alkyl group; preferably, R₇ is a methyl and R₈ is a hydrogen atom or a methyl.
(ii) the compound of formula (IV) below:

   R₉-[O-CH₂-C(H)(R₁₀)]ₚ- (IV),

   in which R₉ represents a C₁-C₄ alkyl group, R₁₀ represents a hydrogen atom or a C₁-C₄ alkyl group and p denotes an integer ranging from 1 to 3; preferably, R₉ is a methyl, ethyl or butyl, R₁₀ is a hydrogen atom or a methyl and p is equal to 1.
(iii) the compound of formula (V) below:

   (R₁₁)₂N-CH₂-C(H)(R₁₂)- (V),

   in which R₁₁ represents a C₁-C₄ alkyl group and R₁₂ represents a hydrogen atom or a C₁-C₄ alkyl group; preferably, R₁₁ is a methyl, ethyl or butyl and R₁₂ is a hydrogen atom or a methyl.
(iv) the compound of formula (VI) below:

   R₁₃-[O-CH₂-C(H)(R₁₄)]_{q}- (VI),

   in which R₁₃ represents a C₁-C₄ alkyl group or a phenyl, R₁₄ represents a hydrogen atom or a C₁-C₄ alkyl group and q denotes an integer ranging from 4 to 30; preferably, R₁₃ is a methyl, ethyl or butyl and R₁₄ is a hydrogen atom or a methyl.

Preferably, R₁ and R₂ independently represent a compound of formula (VI) in which R₁₃ represents a C₁-C₄ alkyl group or a phenyl, preferably a C₁-C₄ alkyl group, more preferentially a methyl, R₁₄ represents a hydrogen atom or a C₁-C₄ alkyl group, preferably a hydrogen atom and q denotes an integer ranging from 4 to 30.

According to an alternative embodiment, R₁ and R₂ are different and one of the radicals R₁ or R₂ represents a compound of formula (IV) as described above and the other radical R₁ or R₂ represents a compound of formula (VI) as described above.

Preferably, in formula (IV), R₉ is a methyl, ethyl or butyl and R₁₀ is a hydrogen atom or a methyl and p is equal to 1.

Preferably, in formula (VI), R₁₃ is a methyl, ethyl or butyl and R₁₄ is a hydrogen atom or a methyl and q denotes an integer ranging from 4 to 30.

According to another alternative embodiment, R₁ and R₂ are identical and represent a compound of formula (VI) in which R₁₃ represents a C₁-C₄ alkyl group or a phenyl, preferably a C₁-C₄ alkyl group, more preferentially a methyl, R₁₄ represents a hydrogen atom or a C₁-C₄ alkyl group, preferably a hydrogen atom and q denotes an integer ranging from 4 to 30.

Preferably, n denotes an integer ranging from 1 to 20, more preferentially from 2 to 20.

Preferably, z denotes an integer ranging from 1 to 20, more preferentially from 2 to 20.

Preferably, w is equal to 1.

Preferably, w is equal to 1, n+z denotes an integer ranging from 4 to 10.

Preferably, L₁ is chosen from a C₁-C₁₈ divalent aliphatic hydrocarbon-based radical such as methylene, ethylene and propylene, a C₃-C₁₅ cycloalkylene radical such as cyclopentylene, cycloheptylene and cyclohexylene, a C₃-C₁₂ heterocycloalkylene group such as imidazolene, pyrrolene and furanylene, or a C₆-C₁₄ arylene group such as phenylene, and mixtures thereof.

For example, L₁ may be chosen from a radical derived from tolylene diisocyanate, hexamethylene diisocyanate, xylylene diisocyanate, 2,2,4-trimethylhexamethylene diisocyanate, 1,12-dodecane diisocyanate, norbornane diisocyanate, 2,4-bis(8-isocyanatooctyl)-1,3-dioctylcyclobutane, 4,4'-dicyclohexylmethane diisocyanate, tetramethylxylylene diisocyanate, isophorone diisocyanate, 1,5-napththylene diisocyanate, 4,4'-diphenylmethane diisocyanate, 4,4'-diphenyldimethylmethane diisocyanate and phenylene diisocyanate, and mixtures thereof.

Preferably, L₁ is chosen from a C₃-C₁₅ cycloalkylene radical or a C₆-C₁₄ arylene group, and mixtures thereof, such as the compounds of formula (VII) below:

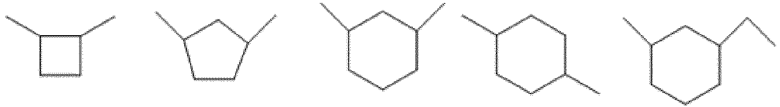

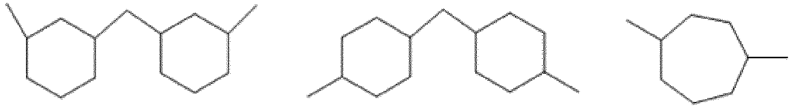

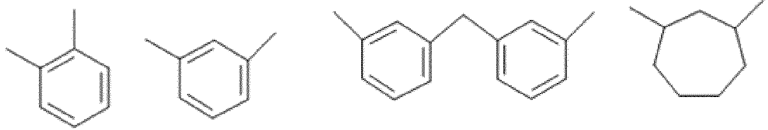

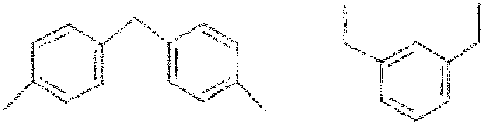

Preferably, L₁ is 4,4-dicyclohexylenemethane corresponding to formula (VIII) below:

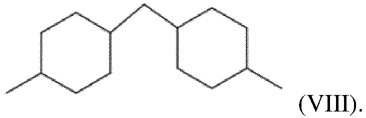

According to another embodiment, when L₁ is a C₆-C₁₄ arylene group, L₁ is not the m-tetramethylxylylene radical represented by formula (IX) below:

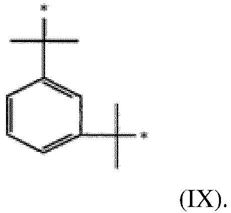

As indicated previously, E independently represents a group chosen from:

-O-R₃-O-; -S-R₄-S-; -R₅-N(R₆)-R₄-N(R₆)-R₅-;

in which R₃ and R₄ independently represent a divalent hydrocarbon-based radical optionally interrupted with one or more heteroatoms;
- R₅ independently represents a covalent bond or a saturated divalent hydrocarbon-based radical, optionally interrupted with one or more heteroatoms; and
- R₆ independently represents a hydrogen atom or a hydrocarbon-based radical, optionally interrupted with one or more heteroatoms.

Preferably, R₃ and R₄ are independently chosen from a C₆-C₁₄ arylene radical such as phenylene, a C₃-C₁₂ cycloalkylene radical such as cyclopropylene and cyclobutylene, a linear or branched C₁-C₁₈ alkylene radical such as methylene and ethylene, optionally interrupted with one or more heteroatoms, and mixtures thereof.

More preferentially, R₃ and R₄ are independently chosen from a linear or branched C₁-C₁₈ alkylene radical such as methylene, butylene, propylene or ethylene, optionally interrupted with one or more heteroatoms.

Preferably, when R₅ is not a covalent bond, R₅ is chosen from a C₆-C₁₄ arylene radical such as phenylene, a C₃-C₁₂ cycloalkylene radical such as cyclopropylene and cyclobutylene, a linear or branched C₁-C₁₈ alkylene radical such as methylene and ethylene, optionally interrupted with one or more heteroatoms, and mixtures thereof.

Preferably, R₆ is chosen from a C₆-C₁₄ arylene radical such as phenylene, a C₃-C₁₂ cycloalkylene radical such as cyclopropylene and cyclobutylene, a linear or branched C₁-C₁₈ alkylene radical such as methylene and ethylene, optionally interrupted with one or more heteroatoms, and mixtures thereof.

Preferably, E represents a group -O-R₃-O- in which R₃ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof.

More preferentially, E represents a group -O-R₃-O- in which R₃ represents a linear or branched C₁-C₁₈ alkylene radical such as methylene, butylene, propylene or ethylene, optionally interrupted with one or more heteroatoms.

According to a particular embodiment, the (poly)carbodiimide compound is a copolymer derived from α-methylstyryl isocyanates of formula (X) below: 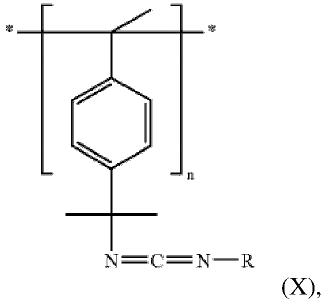 in which R independently represents an alkyl group containing from 1 to 24 carbon atoms, a cycloalkyl group containing from 3 to 24 carbon atoms or an aryl group containing from 6 to 24 carbon atoms, and
n denotes an integer ranging from 2 to 100.

In this embodiment, the term "alkyl group" is as defined previously.

In this embodiment, the term "cycloalkyl group" is as defined previously.

In this embodiment, n may denote an integer ranging from 2 to 50, preferably from 3 to 30 and even more preferentially from 5 to 10.

According to another particular embodiment, the (poly)carbodiimide compound is a compound of formula (XI) below: 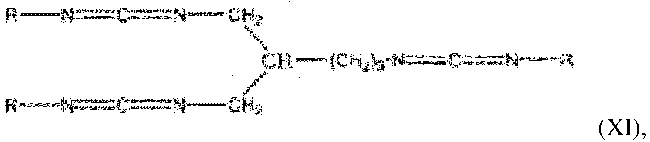 in which R independently represents an alkyl group containing from 1 to 24 carbon atoms, a cycloalkyl group containing from 3 to 24 carbon atoms or an aryl group containing from 6 to 24 carbon atoms.

The "alkyl group", the "cycloalkyl group" and the "aryl group" are as defined previously.

According to a preferred embodiment, the (poly)carbodiimide compound is chosen from the compounds of formula (I) or of formula (II) in which:
- X₁ and X₂ independently represent an oxygen atom;
- R₁ and R₂ are independently chosen from dialkylamino alcohols, alkyl esters of hydroxycarboxylic acid and monoalkyl ethers of (poly)alkylene glycol, in which a hydroxyl group has been removed, and mixtures thereof, preferably monoalkyl ethers of (poly)alkylene glycol, in which a hydroxyl group has been removed, more preferentially the compound of formula (VI) as described previously in which R₁₃ represents a C₁-C₄ alkyl group or a phenyl, preferably a C₁-C₄ alkyl group, more preferentially a methyl, R₁₄ represents a hydrogen atom or a C₁-C₄ alkyl group, preferably a hydrogen atom, and q denotes an integer ranging from 4 to 30;
- n and z, when they are present, denote an integer ranging from 1 to 20, with n+z ≥ 2 and w is equal to 1;
- L₁, when it is present, is chosen from a C₁-C₁₈ divalent aliphatic hydrocarbon-based radical, a C₃-C₁₅ cycloalkylene radical, a C₃-C₁₂ heterocycloalkylene group or a C₆-C₁₄ arylene group, and mixtures thereof, preferably a C₃-C₁₅ cycloalkylene radical;
- A, when it is present, is chosen from a C₁-C₁₈ divalent aliphatic hydrocarbon-based radical, a C₃-C₁₅ cycloalkylene radical, a C₃-C₁₂ heterocycloalkylene group or a C₆-C₁₄ arylene group, and mixtures thereof, preferably a C₃-C₁₅ cycloalkylene radical;
- E, when it is present, independently represents a group chosen from:

   -O-R₃-O-; -S-R₄-S-; -R₅-N(R₆)-R₄-N(R₆)-R₅-;

   in which R₃ and R₄ are independently chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof;
- when R₅ is not a covalent bond, R₅, when it is present, is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof; and
- R₆, when it is present, is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof.

Preferably, the (poly)carbodiimide compound is chosen from the compounds of formula (II) in which:
- X₁ and X₂ independently represent an oxygen atom;
- R₁ and R₂ are independently chosen from dialkylamino alcohols, alkyl esters of hydroxycarboxylic acid and monoalkyl ethers of (poly)alkylene glycol, in which a hydroxyl group has been removed, and mixtures thereof;
- n and z denote an integer ranging from 1 to 20, with n+z ≥ 2 and w is equal to 1;
- L₁ is chosen from a C₁-C₁₈ divalent aliphatic hydrocarbon-based radical, a C₃-C₁₅ cycloalkylene radical, a C₃-C₁₂ heterocycloalkylene group or a C₆-C₁₄ arylene group, and mixtures thereof;
- E independently represents a group chosen from:

   -O-R₃-O-; -S-R₄-S-; -R₅-N(R₆)-R₄-N(R₆)-R₅-;

   in which R₃ and R₄ are independently chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof;
- when R₅ is not a covalent bond, R₅ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof; and
- R₆ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof.

More preferentially, the (poly)carbodiimide compound is chosen from the compounds of formula (II) in which:
- X₁ and X₂ independently represent an oxygen atom;
- R₁ and R₂ are, independently, monoalkyl ethers of (poly)alkylene glycol, in which a hydroxyl group has been removed;
- n and z denote an integer ranging from 1 to 20, with n+z ≥ 2 and w is equal to 1;
- L₁ is a C₃-C₁₅ cycloalkylene radical;
- E independently represents a group chosen from:

   -O-R₃-O-; -S-R₄-S-; -R₅-N(R₆)-R₄-N(R₆)-R₅-;

   in which R₃ and R₄ are independently chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof;
- when R₅ is not a covalent bond, R₅ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof; and
- R₆ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof.

Even more preferentially, the (poly)carbodiimide compound is chosen from the compounds of formula (II) in which:
- X₁ and X₂ independently represent an oxygen atom;
- R₁ and R₂ independently represent the compound of formula (VI) below:

   R₁₃-[O-CH₂-C(H)(R₁₄)]_{q}- (VI),

   in which R₁₃ represents a C₁-C₄ alkyl group or a phenyl, preferably a C₁-C₄ alkyl group, more preferentially a methyl, R₁₄ represents a hydrogen atom or a C₁-C₄ alkyl group, preferably a hydrogen atom and q denotes an integer ranging from 4 to 30;
   - n and z denote an integer ranging from 1 to 20, with n+z ranging from 4 to 10 and w is equal to 1;
   - L₁ is a C₃-C₁₅ cycloalkylene radical such as cyclopentylene, cycloheptylene, cyclohexylene and 4,4-dicyclohexylenemethane; and
   - E represents a group -O-R₃-O- in which R₃ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof.

Even more preferentially, the (poly)carbodiimide compound is chosen from the compounds of formula (II) in which:
- X₁ and X₂ independently represent an oxygen atom;
- R₁ and R₂ independently represent the compound of formula (VI) below:

   R₁₃-[O-CH₂-C(H)(R₁₄)]_{q}- (VI)

   in which R₁₃ represents a C₁-C₄ alkyl group or a phenyl, preferably a C₁-C₄ alkyl group, more preferentially a methyl, R₁₄ represents a hydrogen atom or a C₁-C₄ alkyl group, preferably a hydrogen atom and q denotes an integer ranging from 4 to 30;
- n and z denote an integer ranging from 1 to 20, with n+z ranging from 4 to 10 and w is equal to 1;
- L₁ is a C₃-C₁₅ cycloalkylene radical such as cyclopentylene, cycloheptylene, cyclohexylene and 4,4-dicyclohexylenemethane, preferably 4,4-dicyclohexylenemethane; and
- E represents a group -O-R₃-O- in which R₃ represents a linear or branched C₁-C₁₈ alkylene radical such as methylene, propylene, butylene or ethylene, optionally interrupted with one or more heteroatoms.

According to a preferred embodiment, the (poly)carbodiimide compound is a compound of formula (XII) below: 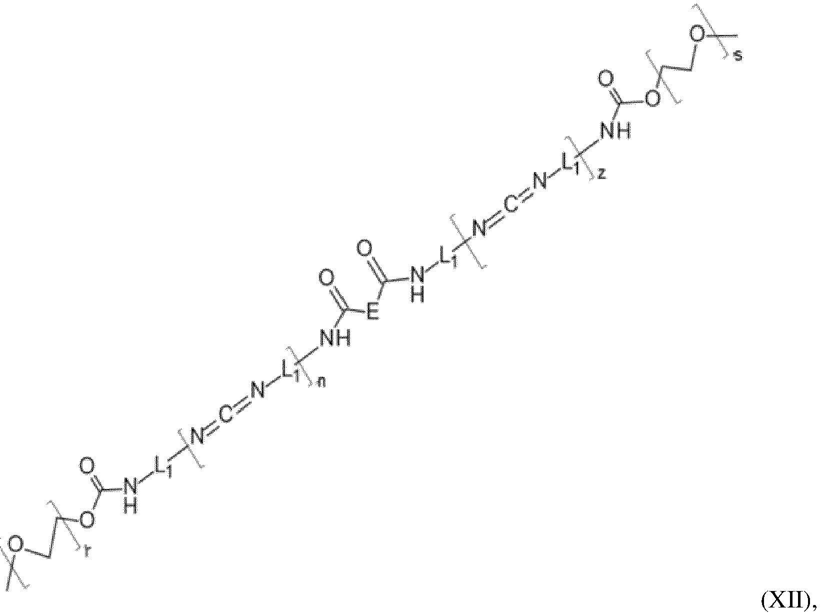 in which L1 is 4,4-dicyclohexylenemethane, n and z denote an integer ranging from 1 to 20, with n+z ranging from 4 to 10, E represents a group -O-R₃-O- in which R₃ represents a linear or branched C₁-C₁₈ alkylene radical such as methylene, propylene, butylene or ethylene, optionally interrupted with one or more heteroatoms, and r and s denote an integer ranging from 4 to 30.

Advantageously, the total amount of the (poly)carbodiimide compound(s) ranges from 0.01% to 20% by weight, preferably from 0.1% to 15% by weight, more preferentially from 0.2% to 10% by weight, even more preferentially from 0.5% to 8% and better still from 1% to 6% by weight, relative to the total weight of composition C.

### Colouring agent

Composition C used in the context of the process according to the invention comprises one or more colouring agents chosen from pigments, direct dyes and mixtures thereof.

Preferably, composition C used in the context of the process according to the invention comprises one or more pigments.

The term "pigment" refers to any pigment that gives colour to keratin materials. Their solubility in water at 25°C and at atmospheric pressure (760 mmHg) is less than 0.05% by weight, and preferably less than 0.01%.

The pigments that may be used are notably chosen from the organic and/or mineral pigments known in the art, notably those described in Kirk-Othmer's *Encyclopedia of Chemical Technology* and in Ullmann's *Encyclopedia of Industrial Chemistry.*

They may be natural, of natural origin, or non-natural.

These pigments may be in pigment powder or paste form. They may be coated or uncoated.

The pigments may be chosen, for example, from mineral pigments, organic pigments, lakes, pigments with special effects such as nacres or glitter flakes, and mixtures thereof.

The pigment may be a mineral pigment. The term "mineral pigment" refers to any pigment that satisfies the definition in Ullmann's encyclopedia in the chapter on inorganic pigments. Among the mineral pigments that are useful in the present invention, mention may be made of iron oxides, chromium oxides, manganese violet, ultramarine blue, chromium hydrate, ferric blue and titanium oxide.

The pigment may be an organic pigment. The term "organic pigment" refers to any pigment that satisfies the definition in Ullmann's Encyclopedia in the chapter on organic pigments.

The organic pigment may notably be chosen from nitroso, nitro, azo, xanthene, pyrene, quinoline, anthraquinone, triphenylmethane, fluorane, phthalocyanine, metal-complex, isoindolinone, isoindoline, quinacridone, perinone, perylene, diketopyrrolopyrrole, indigo, thioindigo, dioxazine, triphenylmethane and quinophthalone compounds.

In particular, the white or coloured organic pigments may be chosen from carmine, carbon black, aniline black, azo yellow, quinacridone, phthalocyanine blue, the blue pigments codified in the Color Index under the references CI 42090, 69800, 69825, 74100, 74160, the yellow pigments codified in the Color Index under the references CI 11680, 11710, 19140, 20040, 21100, 21108, 47000, 47005, the green pigments codified in the Color Index under the references CI 61565, 61570, 74260, the orange pigments codified in the Color Index under the references CI 11725, 45370, 71105, the red pigments codified in the Color Index under the references CI 12085, 12120, 12370, 12420, 12490, 14700, 15525, 15580, 15620, 15630, 15800, 15850, 15865, 15880, 26100, 45380, 45410, 58000, 73360, 73915, 75470, the pigments obtained by oxidative polymerization of indole or phenol derivatives as described in patent FR 2 679 771.

Examples that may also be mentioned include pigment pastes of organic pigments, such as the products sold by the company Hoechst under the names:
- Cosmenyl Yellow 10G: Yellow 3 pigment (CI 11710);
- Cosmenyl Yellow G: Yellow 1 pigment (CI 11680);
- Cosmenyl Orange GR: Orange 43 pigment (CI 71105);
- Cosmenyl Red R: Red 4 pigment (CI 12085);
- Cosmenyl Carmine FB: Red 5 pigment (CI 12490);
- Cosmenyl Violet RL: Violet 23 pigment (CI 51319);
- Cosmenyl Blue A2R: Blue 15.1 pigment (CI 74160);
- Cosmenyl Green GG: Green 7 pigment (CI 74260);
- Cosmenyl Black R: Black 7 pigment (CI 77266).

The pigments in accordance with the invention may also be in the form of composite pigments, as described in patent EP 1 184 426. These composite pigments may notably be composed of particles including an inorganic core, at least one binder for attaching the organic pigments to the core, and at least one organic pigment which at least partially covers the core.

The organic pigment may also be a lake. The term "lake" refers to dyes adsorbed onto insoluble particles, the assembly thus obtained remaining insoluble during use.

The inorganic substrates onto which the dyes are adsorbed are, for example, alumina, silica, calcium sodium borosilicate or calcium aluminium borosilicate and aluminium.

Among the dyes, mention may be made of carminic acid. Mention may also be made of the dyes known under the following names: D&C Red 21 (CI 45 380), D&C Orange 5 (CI 45 370), D&C Red 27 (CI 45 410), D&C Orange 10 (CI 45 425), D&C Red 3 (CI 45 430), D&C Red 4 (CI 15 510), D&C Red 33 (CI 17 200), D&C Yellow 5 (CI 19 140), D&C Yellow 6 (CI 15 985), D&C Green (CI 61 570), D&C Yellow 10 (CI 77 002), D&C Green 3 (CI 42 053), D&C Blue 1 (CI 42 090).

An example of a lake that may be mentioned is the product known under the following name: D&C Red 7 (CI 15 850:1).

The pigment may also be a pigment with special effects. The term "pigments with special effects" means pigments that generally create a coloured appearance (characterized by a certain shade, a certain vivacity and a certain level of luminance) that is non-uniform and that changes as a function of the conditions of observation (light, temperature, angles of observation, etc.). They thereby differ from coloured pigments, which afford a standard uniform opaque, semi-transparent or transparent shade.

Several types of special-effect pigments exist: those with a low refractive index, such as fluorescent or photochromic pigments, and those with a higher refractive index, such as nacres, interference pigments or glitter flakes.

Examples of pigments with special effects that may be mentioned include nacreous pigments such as mica coated with titanium or with bismuth oxychloride, coloured nacreous pigments such as mica covered with titanium and with iron oxides, mica covered with iron oxide, mica covered with titanium and notably with ferric blue or with chromium oxide, mica covered with titanium and with an organic pigment as defined previously, and also nacreous pigments based on bismuth oxychloride. Nacreous pigments that may be mentioned include the nacres Cellini sold by BASF (mica-TiO₂-lake), Prestige sold by Eckart (mica-TiO₂), Prestige Bronze sold by Eckart (mica-Fe₂O₃) and Colorona sold by Merck (mica-TiO₂-Fe₂O₃).

Mention may also be made of the gold-coloured nacres sold notably by the company BASF under the name Brilliant gold 212G (Timica), Gold 222C (Cloisonne), Sparkle gold (Timica), Gold 4504 (Chromalite) and Monarch gold 233X (Cloisonne); the bronze nacres sold notably by the company Merck under the name Bronze fine (17384) (Colorona) and Bronze (17353) (Colorona) and by the company BASF under the name Super bronze (Cloisonne); the orange nacres sold notably by the company BASF under the name Orange 363C (Cloisonne) and Orange MCR 101 (Cosmica) and by the company Merck under the name Passion orange (Colorona) and Matte orange (17449) (Microna); the brown nacres sold notably by the company BASF under the name Nu-antique copper 340XB (Cloisonne) and Brown CL4509 (Chromalite); the nacres with a copper tint sold notably by the company BASF under the name Copper 340A (Timica); the nacres with a red tint sold notably by the company Merck under the name Sienna fine (17386) (Colorona); the nacres with a yellow tint sold notably by the company BASF under the name Yellow (4502) (Chromalite); the red nacres with a gold tint sold notably by the company BASF under the name Sunstone G012 (Gemtone); the pink nacres sold notably by the company BASF under the name Tan opale G005 (Gemtone); the black nacres with a gold tint sold notably by the company BASF under the name Nu antique bronze 240 AB (Timica), the blue nacres sold notably by the company Merck under the name Matte blue (17433) (Microna), the white nacres with a silvery tint sold notably by the company Merck under the name Xirona Silver, and the golden-green pink-orange nacres sold notably by the company Merck under the name Indian summer (Xirona), and mixtures thereof.

Still as examples of nacres, mention may also be made of particles including a borosilicate substrate coated with titanium oxide.

Particles comprising a glass substrate coated with titanium oxide are notably sold under the name Metashine MC1080RY by the company Toyal.

Finally, examples of nacres that may also be mentioned include polyethylene terephthalate glitter flakes, notably those sold by the company Meadowbrook Inventions under the name Silver 1P 0.004X0.004 (silver glitter flakes). It is also possible to envisage multilayer pigments based on synthetic substrates, such as alumina, silica, calcium sodium borosilicate, calcium aluminium borosilicate and aluminium.

The pigments with special effects may also be chosen from reflective particles, i.e. notably from particles whose size, structure, notably the thickness of the layer(s) of which they are made and their physical and chemical nature, and surface state, allow them to reflect incident light. This reflection may, where appropriate, have an intensity sufficient to create at the surface of the composition or of the mixture, when it is applied to the support to be made up, highlight points that are visible to the naked eye, i.e. more luminous points that contrast with their environment making them appear to sparkle.

The reflective particles may be selected so as not to significantly alter the colouring effect generated by the colouring agents with which they are combined, and more particularly so as to optimize this effect in terms of colour rendition. They may more particularly have a yellow, pink, red, bronze, orange, brown, gold and/or coppery colour or tint.

These particles may have varied forms and may notably be in platelet or globular form, in particular in spherical form.

The reflective particles, whatever their form, may or may not have a multilayer structure and, in the case of a multilayer structure, may have, for example, at least one layer of uniform thickness, notably of a reflective material.

When the reflective particles do not have a multilayer structure, they may be composed, for example, of metal oxides, notably titanium or iron oxides obtained synthetically.

When the reflective particles have a multilayer structure, they may include, for example, a natural or synthetic substrate, notably a synthetic substrate at least partially coated with at least one layer of a reflective material, notably of at least one metal or metallic material. The substrate may be made of one or more organic and/or mineral materials.

More particularly, it may be chosen from glasses, ceramics, graphite, metal oxides, aluminas, silicas, silicates, notably aluminosilicates and borosilicates, and synthetic mica, and mixtures thereof, this list not being limiting.

The reflective material may include a layer of metal or of a metallic material.

Reflective particles are notably described in JP-A-09188830, JP-A-10158450, JP-A-10158541, JP-A-07258460 and JP-A-05017710.

Again as an example of reflective particles including a mineral substrate coated with a layer of metal, mention may also be made of particles including a silver-coated borosilicate substrate.

Particles with a silver-coated glass substrate, in the form of platelets, are sold under the name Microglass Metashine REFSX 2025 PS by the company Toyal. Particles with a glass substrate coated with a nickel/chromium/molybdenum alloy are sold under the names Crystal Star GF 550 and GF 2525 by this same company.

Use may also be made of particles comprising a metal substrate, such as silver, aluminium, iron, chromium, nickel, molybdenum, gold, copper, zinc, tin, magnesium, steel, bronze or titanium, said substrate being coated with at least one layer of at least one metal oxide, such as titanium oxide, aluminium oxide, iron oxide, cerium oxide, chromium oxide, silicon oxides and mixtures thereof.

Examples that may be mentioned include aluminium powder, bronze powder or copper powder coated with SiO₂ sold under the name Visionaire by the company Eckart.

Mention may also be made of interference pigments which are not attached to a substrate, such as liquid crystals (Helicones HC from Wacker) or interference holographic glitter flakes (Geometric Pigments or Spectra f/x from Spectratek). Special-effect pigments also comprise fluorescent pigments, whether these are substances that are fluorescent in daylight or that produce an ultraviolet fluorescence, phosphorescent pigments, photochromic pigments, thermochromic pigments and quantum dots, sold, for example, by the company Quantum Dots Corporation.

The variety of pigments that may be used in the present invention makes it possible to obtain a wide range of colours, and also particular optical effects such as metallic effects or interference effects.

The size of the pigment used in the composition according to the present invention is generally between 10 nm and 200 µm, preferably between 20 nm and 80 µm and more preferentially between 30 nm and 50 µm.

The pigments may be dispersed in the composition by means of a dispersant.

The dispersant serves to protect the dispersed particles against agglomeration or flocculation thereof. This dispersant may be a surfactant, an oligomer, a polymer or a mixture of several thereof, bearing one or more functionalities with strong affinity for the surface of the particles to be dispersed. In particular, they may become physically or chemically attached to the surface of the pigments. These dispersants also contain at least one functional group that is compatible with or soluble in the continuous medium. In particular, esters of 12-hydroxystearic acid in particular and of C8 to C20 fatty acid and of polyols such as glycerol or diglycerol are used, such as poly(12-hydroxystearic acid) stearate with a molecular weight of approximately 750 g/mol, such as the product sold under the name Solsperse 21 000 by the company Avecia, polyglyceryl-2 dipolyhydroxystearate (CTFA name) sold under the reference Dehymyls PGPH by the company Henkel, or else polyhydroxystearic acid such as the product sold under the reference Arlacel P100 by the company Uniqema, and mixtures thereof.

As other dispersants that may be used in the compositions of the invention, mention may be made of quaternary ammonium derivatives of polycondensed fatty acids, for instance Solsperse 17 000 sold by the company Avecia, and polydimethylsiloxane/oxypropylene mixtures such as those sold by the company Dow Corning under the references DC2-5185 and DC2-5225 C.

The pigments used in the composition may be surface-treated with an organic agent.

Thus, the pigments surface-treated beforehand that are useful in the context of the invention are pigments which have been completely or partially subjected to a surface treatment of chemical, electronic, electrochemical, mechanochemical or mechanical nature with an organic agent, such as those described notably in Cosmetics and Toiletries, February 1990, Vol. 105, pages 53-64, before being dispersed in the composition in accordance with the invention. These organic agents may be chosen, for example, from waxes, for example carnauba wax and beeswax; fatty acids, fatty alcohols and derivatives thereof, such as stearic acid, hydroxystearic acid, stearyl alcohol, hydroxystearyl alcohol and lauric acid and derivatives thereof; anionic surfactants; lecithins; sodium, potassium, magnesium, iron, titanium, zinc or aluminium salts of fatty acids, for example aluminium stearate or laurate; metal alkoxides; polyethylene; (meth)acrylic polymers, for example polymethyl methacrylates; polymers and copolymers containing acrylate units; alkanolamines; silicone compounds, for example silicones, notably polydimethylsiloxanes; organofluorine compounds, for example perfluoroalkyl ethers; fluorosilicone compounds.

The surface-treated pigments that are useful in the composition may also have been treated with a mixture of these compounds and/or may have undergone several surface treatments.

The surface-treated pigments that are useful in the context of the present invention may be prepared according to surface-treatment techniques that are well known to those skilled in the art, or may be commercially available as is.

Preferably, the surface-treated pigments are coated with an organic layer.

The organic agent with which the pigments are treated may be deposited on the pigments by evaporation of solvent, chemical reaction between the molecules of the surface agent or creation of a covalent bond between the surface agent and the pigments.

The surface treatment may thus be performed, for example, by chemical reaction of a surface agent with the surface of the pigments and creation of a covalent bond between the surface agent and the pigments or the fillers. This method is notably described in patent US 4 578 266.

An organic agent covalently bonded to the pigments will preferably be used.

The agent for the surface treatment may represent from 0.1% to 50% by weight relative to the total weight of the surface-treated pigment, preferably from 0.5% to 30% by weight and even more preferentially from 1% to 20% by weight relative to the total weight of the surface-treated pigment.

Preferably, the surface treatments of the pigments are chosen from the following treatments:
- a PEG-silicone treatment, for instance the AQ surface treatment sold by LCW;
- a methicone treatment, for instance the SI surface treatment sold by LCW;
- a dimethicone treatment, for instance the Covasil 3.05 surface treatment sold by LCW;
- a dimethicone/trimethyl siloxysilicate treatment, for instance the Covasil 4.05 surface treatment sold by LCW;
- a magnesium myristate treatment, for instance the MM surface treatment sold by LCW;
- an aluminium dimyristate treatment, for instance the MI surface treatment sold by Miyoshi;
- a perfluoropolymethyl isopropyl ether treatment, for instance the FHC surface treatment sold by LCW;
- an isostearyl sebacate treatment, for instance the HS surface treatment sold by Miyoshi;
- a perfluoroalkyl phosphate treatment, for instance the PF surface treatment sold by Daito;
- an acrylate/dimethicone copolymer and perfluoroalkyl phosphate treatment, for instance the FSA surface treatment sold by Daito;
- a polymethylhydrogenosiloxane/perfluoroalkyl phosphate treatment, for instance the FS01 surface treatment sold by Daito;
- an acrylate copolymer/dimethicone treatment, for instance the ASC surface treatment sold by Daito;
- an isopropyl titanium triisostearate treatment, for instance the ITT surface treatment sold by Daito;
- an acrylate copolymer treatment, for instance the APD surface treatment sold by Daito;
- a perfluoroalkyl phosphate/isopropyl titanium triisostearate treatment, for instance the PF + ITT surface treatment sold by Daito.

According to a particular embodiment of the invention, the dispersant is present with organic or mineral pigments in submicron-sized particulate form.

The term "submicron-sized" or "submicronic" refers to pigments having a particle size that has been micronized by a micronization method and having a mean particle size of less than a micrometre (µm), in particular between 0.1 and 0.9 µm, and preferably between 0.2 and 0.6 µm.

According to one embodiment, the dispersant and the pigment(s) are present in an amount (dispersant:pigment), according to a weight ratio, of between 1:4 and 4:1, particularly between 1.5:3.5 and 3.5:1 or better still between 1.75:3 and 3:1.

The dispersant(s) may thus have a silicone backbone, such as silicone polyether and dispersants of amino silicone type other than the amino silicones mentioned hereinbelow. Among the suitable dispersants that may be mentioned are:
- amino silicones, i.e. silicones comprising one or more amine groups such as those sold under the names and references: BYK LPX 21879 by BYK, GP-4, GP-6, GP-344, GP-851, GP-965, GP-967 and GP-988-1, sold by Genesee Polymers,
- silicone acrylates such as Tego^{®} RC 902, Tego^{®} RC 922, Tego^{®} RC 1041, and Tego^{®} RC 1043, sold by Evonik,
- polydimethylsiloxane (PDMS) silicones bearing carboxyl groups such as X-22162 and X-22370 by Shin-Etsu, epoxy silicones such as GP-29, GP-32, GP-502, GP-504, GP-514, GP-607, GP-682, and GP-695 by Genesee Polymers, or Tego^{®} RC 1401, Tego^{®} RC 1403, Tego^{®} RC 1412 by Evonik.

According to a particular embodiment, the dispersant(s) are of amino silicone type other than the amino silicones mentioned hereinbelow and are cationic.

Preferably, the pigment(s) are chosen from mineral, mixed mineral-organic or organic pigments.

In one variant of the invention, the pigment(s) are organic pigments, preferentially organic pigments surface-treated with an organic agent chosen from silicone compounds. In another variant of the invention, the pigment(s) are mineral pigments.

Preferably, the pigment(s) are chosen from iron oxides, notably red, brown or black iron oxides. As an example of an iron oxide, mention may be made of the iron oxide sold by the company Sun Chemical under the name SunPuro^{®} Red Iron Oxide.

### Direct dye

Composition C used in the context of the process according to the invention may comprise one or more direct dyes.

The term "direct dye" means natural and/or synthetic dyes, other than oxidation dyes. These are dyes which will spread superficially over the fibre.

They may be ionic or nonionic, preferably cationic or nonionic.

Examples of suitable direct dyes that may be mentioned include azo direct dyes; (poly)methine dyes such as cyanines, hemicyanines and styryls; carbonyl dyes; azine dyes; nitro(hetero)aryl dyes; tri(hetero)arylmethane dyes; porphyrin dyes; phthalocyanine dyes and natural direct dyes, alone or in the form of mixtures.

The direct dyes are preferably cationic direct dyes. Mention may be made of the hydrazono cationic dyes of formulae (XIII) and (XIV) and the azo cationic dyes (XV) and (XVI) below:

Het⁺-N(Ra)-N=C(Rb)-Ar, Q- (XIII),

Het⁺-C(Ra)=N-N(Rb)-Ar, Q (XIV),

Het⁺-N=N-Ar, Q- (XV),

Ar⁺-N=N-Ar", Q- (XVI),

in which formulae (XIII) to (XVI):
- Het⁺ represents a cationic heteroaryl radical, preferentially bearing an endocyclic cationic charge, such as imidazolium, indolium or pyridinium, which is optionally substituted, preferentially with at least one (C₁-C₈)alkyl group such as methyl;
- Ar⁺ represents an aryl radical, such as phenyl or naphthyl, bearing an exocyclic cationic charge, preferentially ammonium, particularly tri(C₁-C₈)alkylammonium, such as trimethylammonium;
- Ar represents an aryl group, notably phenyl, which is optionally substituted, preferentially with one or more electron-donating groups such as i) optionally substituted (C₁-C₈)alkyl, ii) optionally substituted (C₁-C₈)alkoxy, iii) (di)(C₁-Cs)(alkyl)amino optionally substituted on the alkyl group(s) with a hydroxyl group, iv) aryl(C₁-C₈)alkylamino, v) optionally substituted N-(C₁-C₈)alkyl-N-aryl(C₁-Cs)alkylamino or alternatively Ar represents a julolidine group;
- Ar" represents an optionally substituted (hetero)aryl group, such as phenyl or pyrazolyl, which are optionally substituted, preferentially with one or more (C₁-C₈)alkyl, hydroxyl, (di)(C₁-C₈)(alkyl)amino, (C₁-C₈)alkoxy or phenyl groups;
- Ra and Rb, which may be identical or different, represent a hydrogen atom or a (C₁-C₈)alkyl group, which is optionally substituted, preferentially with a hydroxyl group;
   or else the substituent Ra with a substituent of Het+ and/or Rb with a substituent of Ar form, together with the atoms that bear them, a (hetero)cycloalkyl; in particular, Ra and Rb represent a hydrogen atom or a (C₁-C₄)alkyl group optionally substituted with a hydroxyl group;
- Q- represents an organic or mineral anionic counterion, such as a halide or an alkyl sulfate.

In particular, mention may be made of the azo and hydrazono direct dyes bearing an endocyclic cationic charge of formulae (XIII) to (XVI) as defined previously, more particularly, the cationic direct dyes bearing an endocyclic cationic charge described in patent applications WO 95/15144, WO 95/01772 and EP 714 954, preferentially the following direct dyes: 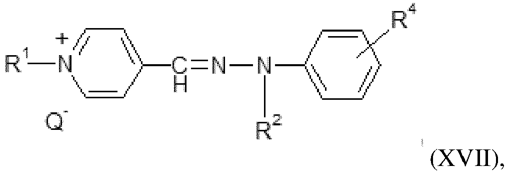 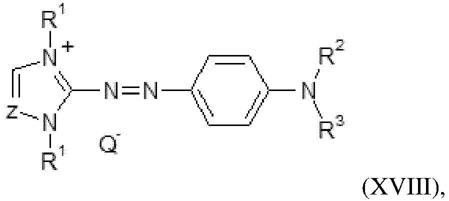 in which formulae (XVII) and (XVIII):
- R¹ represents a (C₁-C₄)alkyl group such as methyl;
- R² and R³, which may be identical or different, represent a hydrogen atom or a (C₁-C₄)alkyl group, such as methyl; and
- R⁴ represents a hydrogen atom or an electron-donating group such as optionally substituted (C₁-C₈)alkyl, optionally substituted (C₁-C₈)alkoxy, or (di)(C₁-C₈)(alkyl)amino optionally substituted on the alkyl group(s) with a hydroxyl group; in particular, R₄ is a hydrogen atom;
- Z represents a CH group or a nitrogen atom, preferentially CH;
- Q- is an anionic counterion as defined previously, in particular a halide, such as chloride, or an alkyl sulfate, such as methyl sulfate or mesyl.

In particular, the dyes of formulae (XV) and (XVI) are chosen from Basic Red 51, Basic Yellow 87 and Basic Orange 31 or derivatives thereof with Q' being an anionic counterion as defined previously, particularly a halide such as chloride, or an alkyl sulfate such as methyl sulfate or mesyl.

The direct dyes may be chosen from anionic direct dyes. The anionic direct dyes of the invention are dyes commonly referred to as "acid" direct dyes owing to their affinity for alkaline substances. The term "anionic direct dye" means any direct dye including in its structure at least one CO₂R or SO₃R substituent with R denoting a hydrogen atom or a cation originating from a metal or an amine, or an ammonium ion. The anionic dyes may be chosen from direct nitro acid dyes, azo acid dyes, azine acid dyes, triarylmethane acid dyes, indoamine acid dyes, anthraquinone acid dyes, indigoid dyes and natural acid dyes.

As acid dyes that are useful for the invention, mention may be made of the dyes of formulae (XIX), (XIX'), (XX), (XX'), (XXI), (XXI'), (XXII), (XXII'), (XXIII), (XXIV), (XXV) and (XXVI) below:
a) the diaryl anionic azo dyes of formula (XIX) or (XIX'): 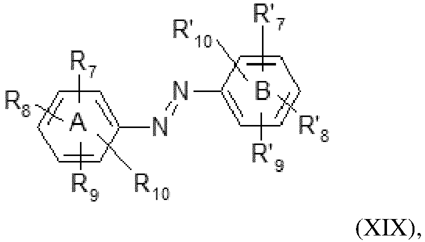 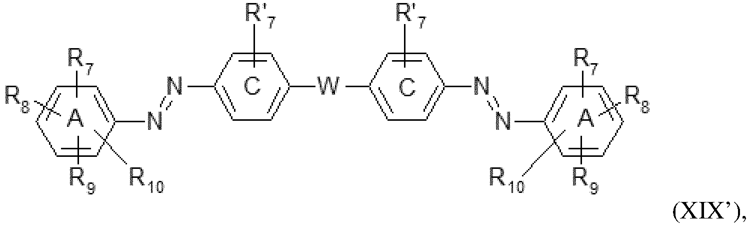 in which formulae (XIX) and (XIX'):
   - R₇, R₈, R₉, R₁₀, R'₇, R'₈, R'₉ and R'₁₀, which may be identical or different, represent a hydrogen atom or a group chosen from:
      - alkyl;
      - alkoxy, alkylthio;
      - hydroxyl, mercapto;
      - nitro, nitroso;
      - R°-C(X)-X'-, R°-X'-C(X)-, R°-X'-C(X)-X''- with R° representing a hydrogen atom or an alkyl or aryl group; X, X' and X'', which may be identical or different, representing an oxygen or sulfur atom, or NR with R representing a hydrogen atom or an alkyl group;
      - (O)₂S(O⁻)-, M⁺ with M⁺ representing a hydrogen atom or a cationic counterion;
      - (O)CO⁻-, M⁺ with M⁺ as defined previously;
      - R"-S(O)₂-, with R" representing a hydrogen atom or an alkyl group, an aryl, (di)(alkyl)amino or aryl(alkyl)amino group; preferentially a phenylamino or phenyl group;
      - R‴-S(O)₂-X'- with R‴ representing an optionally substituted alkyl or aryl group, X' as defined previously;
      - (di)(alkyl)amino;
      - aryl(alkyl)amino optionally substituted with one or more groups chosen from i) nitro; ii) nitroso; iii) (O)₂S(O⁻)-, M⁺ and iv) alkoxy with M⁺ as defined previously;
      - optionally substituted heteroaryl; preferentially a benzothiazolyl group;
      - cycloalkyl, notably cyclohexyl;
      - Ar-N=N- with Ar representing an optionally substituted aryl group; preferentially a phenyl optionally substituted with one or more alkyl, (O)₂S(O⁻)-, M⁺ or phenylamino groups;
      - or alternatively two contiguous groups R₇ with R₈ or R₈ with R₉ or R₉ with R₁₀ together form a fused benzo group A'; and R'₇ with R'₈ or R'₈ with R'₉ or R'₉ with R'₁₀ together form a fused benzo group B'; with A' and B' optionally substituted with one or more groups chosen from i) nitro; ii) nitroso; iii) (O)₂S(O⁻)-, M⁺; iv) hydroxyl; v) mercapto; vi) (di)(alkyl)amino; vii) R°-C(X)-X'-; viii) R°-X'-C(X)-; ix) R°-X'-C(X)-X" -; x) Ar-N=N- and xi) optionally substituted aryl(alkyl)amino; with M⁺, R°, X, X', X" and Ar as defined previously;
      - W represents a sigma bond σ, an oxygen or sulfur atom, or a divalent radical i) -NR- with R as defined previously, or ii) methylene -C(Ra)(Rb)- with Ra and Rb, which may be identical or different, representing a hydrogen atom or an aryl group, or alternatively Ra and Rb form, together with the carbon atom that bears them, a spiro cycloalkyl; preferentially, W represents a sulfur atom or Ra and Rb together form a cyclohexyl;
   it being understood that formulae (XIX) and (XIX') comprise at least one sulfonate radical (O)₂S(O⁻)-, M⁺ or one carboxylate radical (O)CO⁻-, M⁺ on one of the rings A, A', B, B' or C; preferentially sodium sulfonate.

As examples of dyes of formula (XIX), mention may be made of: Acid Red 1, Acid Red 4, Acid Red 13, Acid Red 14, Acid Red 18, Acid Red 27, Acid Red 28, Acid Red 32, Acid Red 33, Acid Red 35, Acid Red 37, Acid Red 40, Acid Red 41, Acid Red 42, Acid Red 44, Pigment Red 57, Acid Red 68, Acid Red 73, Acid Red 135, Acid Red 138, Acid Red 184, Food Red 1, Food Red 13, Acid Orange 6, Acid Orange 7, Acid Orange 10, Acid Orange 19, Acid Orange 20, Acid Orange 24, Yellow 6, Acid Yellow 9, Acid Yellow 36, Acid Yellow 199, Food Yellow 3, Acid Violet 7, Acid Violet 14, Acid Blue 113, Acid Blue 117, Acid Black 1, Acid Brown 4, Acid Brown 20, Acid Black 26, Acid Black 52, Food Black 1, Food Black 2, Food Yellow 3 or Sunset Yellow;
and, as examples of dyes of formula (XIX'), mention may be made of: Acid Red 111, Acid Red 134, Acid Yellow 38;
b) the pyrazolone anionic azo dyes of formulae (XX) and (XX'): 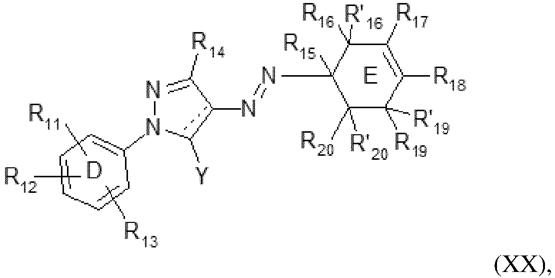 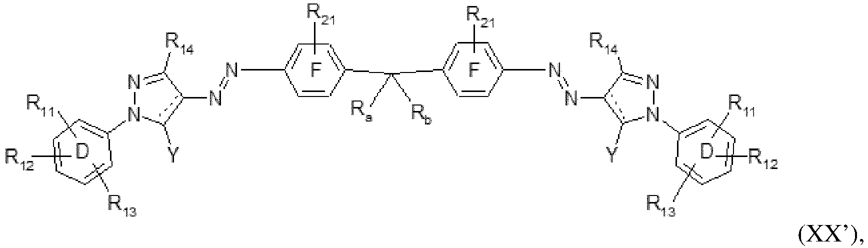
in which formulae (XX) and (XX'):
   - R₁₁, R₁₂ and R₁₃, which may be identical or different, represent a hydrogen or halogen atom, an alkyl group or -(O)₂S(O⁻), M⁺ with M⁺ as defined previously;
   - R₁₄ represents a hydrogen atom, an alkyl group or a group -C(O)O⁻, M⁺ with M⁺ as defined previously;
   - R₁₅ represents a hydrogen atom;
   - R₁₆ represents an oxo group, in which case R'₁₆ is absent, or alternatively R₁₅ with R₁₆ together form a double bond;
   - R₁₇ and R₁₈, which may be identical or different, represent a hydrogen atom, or a group chosen from:
      - (O)₂S(O⁻)-, M⁺ with M⁺ as defined previously;
      - Ar-O-S(O)₂- with Ar representing an optionally substituted aryl group; preferentially a phenyl optionally substituted with one or more alkyl groups;
      - R₁₉ and R₂₀ together form either a double bond, or a benzo group D', which is optionally substituted;
      - R'₁₆, R'₁₉ and R'₂₀, which may be identical or different, represent a hydrogen atom or an alkyl or hydroxyl group;
      - R₂₁ represents a hydrogen atom or an alkyl or alkoxy group;
      - Ra and Rb, which may be identical or different, are as defined previously; preferentially, Ra represents a hydrogen atom and Rb represents an aryl group;
      - Y represents either a hydroxyl group or an oxo group;
      - - - - represents a single bond when Y is an oxo group; and represents a double bond when Y represents a hydroxyl group;
it being understood that formulae (XX) and (XX') comprise at least one sulfonate radical (O)₂S(O⁻)-, M⁺ or one carboxylate radical -C(O)O⁻, M⁺ on one of the rings D or E; preferentially sodium sulfonate.

As examples of dyes of formula (XX), mention may be made of: Acid Red 195, Acid Yellow 23, Acid Yellow 27, Acid Yellow 76, and as examples of dyes of formula (XX'), mention may be made of: Acid Yellow 17;
c) the anthraquinone dyes of formulae (XXI) and (XXI'): 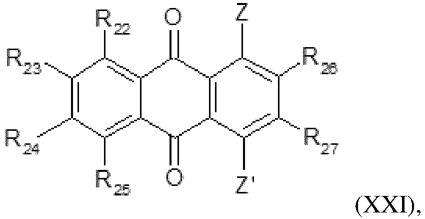 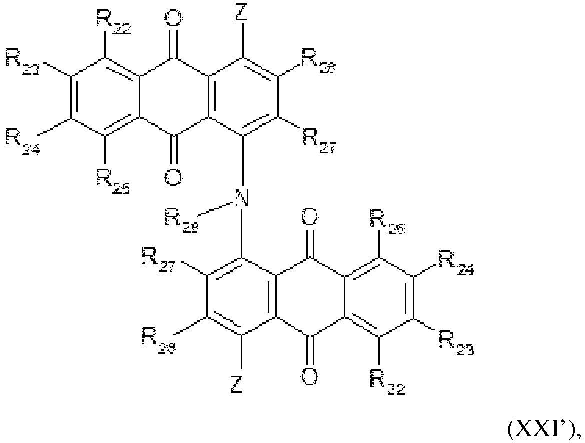
in which formulae (XXI) and (XXI'):
   - R₂₂, R₂₃, R₂₄, R₂₅, R₂₆ and R₂₇, which may be identical or different, represent a hydrogen or halogen atom, or a group chosen from:
      - alkyl;
      - hydroxyl, mercapto;
      - alkoxy, alkylthio;
      - optionally substituted aryloxy or arylthio, preferentially substituted with one or more groups chosen from alkyl and (O)₂S(O⁻)-, M⁺ with M⁺ as defined previously;
      - aryl(alkyl)amino optionally substituted with one or more groups chosen from alkyl and (O)₂S(O⁻)-, M⁺ with M⁺ as defined previously;
      - (di)(alkyl)amino;
      - (di)(hydroxyalkyl)amino;
      - (O)₂S(O⁻)-, M⁺ with M⁺ as defined previously;
      - Z' represents a hydrogen atom or a group NR₂₈R₂₉ with R₂₈ and R₂₉, which may be identical or different, representing a hydrogen atom or a group chosen from:
      - alkyl;
      - polyhydroxyalkyl such as hydroxyethyl;
      - aryl optionally substituted with one or more groups, particularly i) alkyl such as methyl, n-dodecyl, n-butyl; ii) (O)₂S(O⁻)-, M⁺ with M⁺ as defined previously; iii) R°-C(X)-X'-, R°-X'-C(X)-, R°-X'-C(X)-X"- with R°, X, X' and X" as defined previously, preferentially R° represents an alkyl group;
      - cycloalkyl, notably cyclohexyl;
      - Z represents a group chosen from hydroxyl and NR'₂₈R'₂₉ with R'₂₈ and R'₂₉, which may be identical or different, representing the same atoms or groups as R₂₈ and R₂₉ as defined previously;
it being understood that formulae (XXI) and (XXI') comprise at least one sulfonate radical (O)₂S(O⁻)-, M⁺ or one carboxylate radical -C(O)O⁻, M⁺; preferentially sodium sulfonate.

As examples of dyes of formula (XXI), mention may be made of: Acid Blue 25, Acid Blue 43, Acid Blue 62, Acid Blue 78, Acid Blue 129, Acid Blue 138, Acid Blue 140, Acid Blue 251, Acid Green 25, Acid Green 41, Acid Violet 42, Acid Violet 43, Mordant Red 3; EXT Violet No. 2; and, as an example of a dye of formula (XXI'), mention may be made of: Acid Black 48;
d) the nitro dyes of formulae (XXII) and (XXII'): 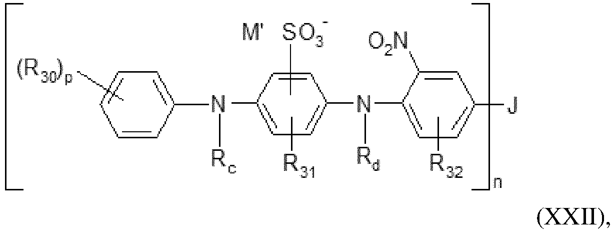 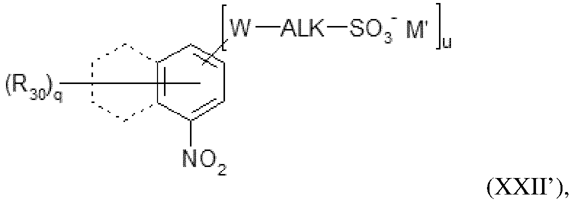
in which formulae (XXII) and (XXII'):
   - R₃₀, R₃₁ and R₃₂, which may be identical or different, represent a hydrogen or halogen atom, or a group chosen from:
      - alkyl;
      - alkoxy optionally substituted with one or more hydroxyl groups, alkylthio optionally substituted with one or more hydroxyl groups;
      - hydroxyl, mercapto;
      - nitro, nitroso;
      - polyhaloalkyl;
      - R°-C(X)-X'-, R°-X'-C(X)-, R°-X'-C(X)-X''- with R°, X, X' and X'' as defined previously;
      - (O)₂S(O⁻)-, M⁺ with M⁺ as defined previously;
      - (O)CO⁻-, M⁺ with M⁺ as defined previously;
      - (di)(alkyl)amino;
      - (di)(hydroxyalkyl)amino;
      - heterocycloalkyl such as piperidino, piperazino or morpholino; in particular, R₃₀, R₃₁ and R₃₂ represent a hydrogen atom;
      - Rc and Rd, which may be identical or different, represent a hydrogen atom or an alkyl group;
      - W is as defined previously; W particularly represents an -NH- group;
      - ALK represents a linear or branched divalent C₁-C₆ alkylene group; in particular, ALK represents a -CH₂-CH₂- group;
      - n is 1 or 2;
      - p represents an integer inclusively between 1 and 5;
      - q represents an integer inclusively between 1 and 4;
      - u is 0 or 1;
      - when n is 1, J represents a nitro or nitroso group; particularly nitro;
      - when n is 2, J represents an oxygen or sulfur atom, or a divalent radical - S(O)ₘ- with m representing an integer 1 or 2; preferentially, J represents an -SO₂-radical;
      - M' represents a hydrogen atom or a cationic counterion; 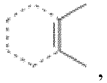 which may be present or absent, represents a benzo group optionally substituted with one or more groups R₃₀ as defined previously;
it being understood that formulae (XXII) and (XXII') comprise at least one sulfonate radical (O)₂S(O⁻)-, M⁺ or one carboxylate radical -C(O)O⁻, M⁺; preferentially sodium sulfonate.

As examples of dyes of formula (XXII), mention may be made of: Acid Brown 13 and Acid Orange 3; as examples of dyes of formula (XXII'), mention may be made of: Acid Yellow 1, the sodium salt of 2,4-dinitro-1-naphthol-7-sulfonic acid, 2-piperidino-5-nitrobenzenesulfonic acid, 2-(4'-N,N-(2"-hydroxyethyl)amino-2'-nitro)anilineethanesulfonic acid, 4-β-hydroxyethylamino-3-nitrobenzenesulfonic acid; EXT D&C Yellow 7;
e) the triarylmethane dyes of formula (XXIII): 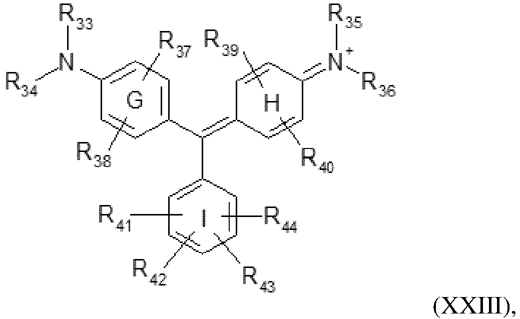
in which formula (XXIII):
   - R₃₃, R₃₄, R₃₅ and R₃₆, which may be identical or different, represent a hydrogen atom or a group chosen from alkyl, optionally substituted aryl and optionally substituted arylalkyl; particularly an alkyl and benzyl group optionally substituted with a group (O)ₘS(O⁻)-, M⁺ with M⁺ and m as defined previously;
   - R₃₇, R₃₈, R₃₉, R₄₀, R₄₁, R₄₂, R₄₃ and R₄₄, which may be identical or different, represent a hydrogen atom or a group chosen from:
      - alkyl;
      - alkoxy, alkylthio;
      - (di)(alkyl)amino;
      - hydroxyl, mercapto;
      - nitro, nitroso;
      - R°-C(X)-X'-, R°-X'-C(X)-, R°-X'-C(X)-X"- with R° representing a hydrogen atom or an alkyl or aryl group; X, X' and X", which may be identical or different, representing an oxygen or sulfur atom, or NR with R representing a hydrogen atom or an alkyl group;
      - (O)₂S(O⁻)-, M⁺ with M⁺ representing a hydrogen atom or a cationic counterion;
      - (O)CO⁻-, M⁺ with M⁺ as defined previously;
      - or alternatively two contiguous groups R₄₁ with R₄₂ or R₄₂ with R₄₃ or R₄₃ with R₄₄ together form a fused benzo group: I'; with I' optionally substituted with one or more groups chosen from i) nitro; ii) nitroso; iii) (O)₂S(O⁻)-, M⁺; iv) hydroxyl; v) mercapto; vi) (di)(alkyl)amino; vii) R°-C(X)-X'-; viii) R°-X'-C(X)- and ix) R°-X'-C(X)-X"-; with M⁺, R°, X, X' and X" as defined previously;
in particular, R₃₇ to R₄₀ represent a hydrogen atom, and R₄₁ to R₄₄, which may be identical or different, represent a hydroxyl group or (O)₂S(O⁻)-, M⁺; and when R₄₃ with R₄₄ together form a benzo group, it is preferentially substituted with an (O)₂S(O⁻)- group;
it being understood that at least one of the rings G, H, I or I' comprises at least one sulfonate radical (O)₂S(O⁻)- or one carboxylate radical -C(O)O⁻; preferentially sulfonate.

As examples of dyes of formula (XXIII), mention may be made of: Acid Blue 1; Acid Blue 3; Acid Blue 7, Acid Blue 9; Acid Violet 49; Acid Green 3; Acid Green 5 and Acid Green 50.
f) the xanthene-based dyes of formula (XXIV): 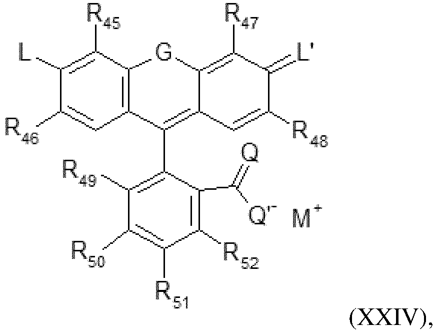
in which formula (XXIV):
   - R₄₅, R₄₆, R₄₇ and R₄₈, which may be identical or different, represent a hydrogen or halogen atom;
   - R₄₉, R₅₀, R₅₁ and R₅₂, which may be identical or different, represent a hydrogen or halogen atom, or a group chosen from:
      - alkyl;
      - alkoxy, alkylthio;
      - hydroxyl, mercapto;
      - nitro, nitroso;
      - (O)₂S(O⁻)-, M⁺ with M⁺ representing a hydrogen atom or a cationic counterion;
      - (O)CO⁻-, M⁺ with M⁺ as defined previously;
particularly, R₄₉, R₅₀, R₅₁ and R₅₂ represent a hydrogen or halogen atom;
   - G represents an oxygen or sulfur atom or a group NRe with Re as defined previously; in particular, G represents an oxygen atom;
   - L represents an alkoxide O⁻, M⁺; a thioalkoxide S⁻, M⁺ or a group NRf, with Rf representing a hydrogen atom or an alkyl group, and M⁺ as defined above; M⁺ is particularly sodium or potassium;
   - L' represents an oxygen or sulfur atom or an ammonium group: N⁺RfRg, with Rf and Rg, which may be identical or different, representing a hydrogen atom or an optionally substituted alkyl or aryl group; L' particularly represents an oxygen atom or a phenylamino group optionally substituted with one or more alkyl or (O)ₘS(O⁻)-, M⁺ groups with m and M⁺ as defined previously;
   - Q and Q', which may be identical or different, represent an oxygen or sulfur atom; particularly, Q and Q' represent an oxygen atom;
   - M⁺ is as defined previously.

As examples of dyes of formula (XXIV), mention may be made of: Acid Yellow 73; Acid Red 51; Acid Red 52; Acid Red 87; Acid Red 92; Acid Red 95; Acid Violet 9;
g) the indole-based dyes of formula (XXV): 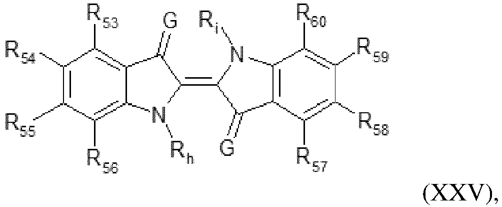
in which formula (XXV):
   - R₅₃, R₅₄, R₅₅, R₅₆, R₅₇, R₅₈, R₅₉ and R₆₀, which may be identical or different, represent a hydrogen atom or a group chosen from:
      - alkyl;
      - alkoxy, alkylthio;
      - hydroxyl, mercapto;
      - nitro, nitroso;
      - R°-C(X)-X'-, R°-X'-C(X)-, R°-X'-C(X)-X''- with R° representing a hydrogen atom or an alkyl or aryl group; X, X' and X'', which may be identical or different, representing an oxygen or sulfur atom, or NR with R representing a hydrogen atom or an alkyl group;
      - (O)₂S(O⁻)-, M⁺ with M⁺ representing a hydrogen atom or a cationic counterion;
      - (O)CO⁻-, M⁺ with M⁺ as defined previously;
      - G represents an oxygen or sulfur atom or a group NRe with Re as defined previously; in particular, G represents an oxygen atom;
      - Ri and Rh, which may be identical or different, represent a hydrogen atom or an alkyl group;
it being understood that formula (XXIII) comprises at least one sulfonate radical (O)₂S(O⁻)-, M⁺ or one carboxylate radical -C(O)O⁻, M⁺; preferentially sodium sulfonate.

As examples of dyes of formula (XXV), mention may be made of: Acid Blue 74;
h) the quinoline-based dyes of formula (XXVI): 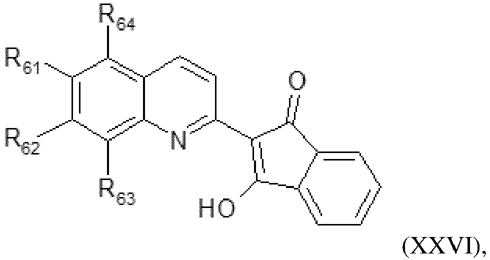
in which formula (XXVI):
   - R₆₁ represents a hydrogen or halogen atom or an alkyl group;
   - R₆₂, R₆₃ and R₆₄, which may be identical or different, represent a hydrogen atom or a group (O)₂S(O⁻)-, M⁺ with M⁺ representing a hydrogen atom or a cationic counterion;
or alternatively R₆₁ with R₆₂, or R₆₁ with R₆₄, together form a benzo group optionally substituted with one or more groups (O)₂S(O⁻)-, M⁺ with M⁺ representing a hydrogen atom or a cationic counterion;
it being understood that formula (XXVI) comprises at least one sulfonate radical (O)₂S(O⁻)-, M⁺, preferentially sodium sulfonate.

As examples of dyes of formula (XXVI), mention may be made of: Acid Yellow 2, Acid Yellow 3 and Acid Yellow 5.

Among the natural direct dyes that may be used according to the invention, mention may be made of lawsone, juglone, alizarin, purpurin, carminic acid, kermesic acid, purpurogallin, protocatechaldehyde, indigo, isatin, curcumin, spinulosin, apigenidin and orceins. Use may also be made of extracts or decoctions containing these natural dyes and particularly henna-based poultices or extracts.

Preferably, the direct dyes are chosen from anionic direct dyes.

The colouring agent(s) may be present in a total amount ranging from 0.001% to 20% by weight and preferably from 0.005% to 15% by weight relative to the total weight of composition C; preferably, the colouring agents are chosen from pigments.

The pigment(s) may be present in a total amount ranging from 0.05% to 20% by weight, preferably from 0.1% to 15% by weight and better still from 0.5% to 10% by weight, relative to the total weight of composition C.

The direct dye(s) may be present in a total amount ranging from 0.001% to 10% by weight and preferably from 0.005% to 5% by weight relative to the total weight of composition C.

### Compound containing at least one carboxylic function

Composition C used in the context of the process according to the invention may also comprise at least one compound containing at least one carboxylic function.

Preferably, the compound containing at least one carboxylic function is chosen from silicone compounds comprising at least one carboxylic group, polyurethanes, acrylic polymers and mixtures thereof.

### Polyurethanes and acrylic polymers

According to a preferred embodiment, composition C comprises at least one compound containing at least one carboxylic function, chosen from polyurethanes, acrylic polymers and mixtures thereof.

Preferably, the compound(s) containing at least one carboxylic function are in the form of aqueous dispersions of particles of polymer(s) chosen from polyurethanes, acrylic polymers and mixtures thereof.

Preferably, composition C comprises at least one compound containing at least one carboxylic function in the form of aqueous dispersions of particles of polyurethanes, acrylic polymers and mixtures thereof.

The dispersion(s) may be simple dispersions in the aqueous medium of the cosmetic composition. As a particular case of dispersions, mention may be made of latices.

The aqueous dispersion(s) of polymer particles may be chosen from aqueous dispersions of polyurethane particles.

More particularly, the polyurethane(s) present in the aqueous dispersions used in the present invention result from the reaction of:
- a prepolymer of formula (A) below: 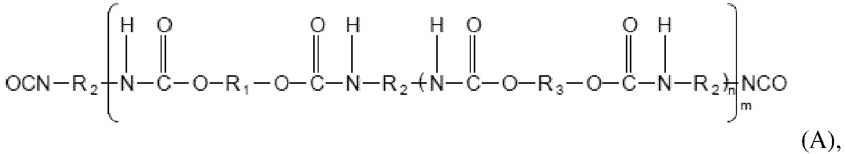 in which:
   - R₁ represents a divalent radical of a dihydroxylated compound;
   - R₂ represents a radical of an aliphatic or cycloaliphatic polyisocyanate;
   - R₃ represents a radical of a low molecular weight diol, optionally substituted with one or more ionic groups;
   - n represents an integer ranging from 1 to 5, and
   - m is greater than 1;
      - at least one chain extender according to formula (B) below:

         H₂N-R₄-NH₂ (B),

         in which R₄ represents an alkylene or alkylene oxide radical which is not substituted with one or more ionic or potentially ionic groups; and
      - at least one chain extender according to formula (C) below:

         H₂N-R₅-NH₂ (C),

         in which R₅ represents an alkylene radical substituted with one or more ionic or potentially ionic groups.

Among the dihydroxylated compounds that may be used according to the present invention, mention may be made notably of the compounds containing two hydroxyl groups and having a number-average molecular weight from about 700 to about 16 000, and preferably from about 750 to about 5000. By way of example of dihydroxylated compounds having a high molecular weight, mention may be made of polyol polyesters, polyol polyethers, polyhydroxylated polycarbonates, polyhydroxylated polyacetates, polyhydroxylated polyacrylates, polyhydroxylated amide polyesters, polyhydroxylated polyalkadienes, polyhydroxylated polythioethers, and mixtures thereof. Preferably, the hydroxylated compounds are chosen from polyol polyesters, polyol polyethers, polyhydroxylated polycarbonates, and mixtures thereof.

The polyisocyanates that may be used according to the present invention are notably chosen from organic diisocyanates with a molecular weight from about 112 to 1000, and preferably from about 140 to 400.

Preferably, the polyisocyanates are chosen from diisocyanates and more particularly from those represented by the general formula R₂(NCO)₂, in which R₂ represents a divalent aliphatic hydrocarbon-based group containing from 4 to 18 carbon atoms, a divalent cycloaliphatic hydrocarbon-based group containing from 5 to 15 carbon atoms, a divalent araliphatic hydrocarbon-based group containing from 7 to 15 carbon atoms or a divalent aromatic hydrocarbon-based group containing from 6 to 15 carbon atoms.

Preferably, R₂ represents an organic diisocyanate. As examples of organic diisocyanates, the following may notably be chosen: tetramethylene diisocyanate, 1,6-hexamethylene diisocyanate, dodecamethylene diisocyanate, 1,3-diisocyanatocyclohexane, 1,4-diisocyanatocyclohexane, 3-isocyanatomethyl-3,5,5-trimethylcyclohexane isocyanate (isophorone diisocyanate or IPDI), bis(4-isocyanatocyclohexyl)methane, 1,3-bis(isocyanatomethyl)cyclohexane, 1,4-bis(isocyanatomethyl)cyclohexane, bis(4-isocyanato-3-methylcyclohexyl)methane, isomers of toluene diisocyanate (TDI) such as toluene 2,4-diisocyanate, toluene 2,6-diisocyanate and mixtures thereof, hydrogenated toluene diisocyanate, diphenylmethane 4,4'-diisocyanate and mixtures with its isomers diphenylmethane 2,4-diisocyanate and optionally diphenylmethane 2,2'-diisocyanate, naphthalene 1,5-diisocyanate, and mixtures thereof.

Preferably, the diisocyanates are aliphatic and cycloaliphatic diisocyanates, and are more preferentially chosen from 1,6-hexamethylene diisocyanate, 3-isocyanatomethyl-3,5,5-trimethylcyclohexane isocyanate, and mixtures thereof.

According to the present invention, the term *"low molecular weight diol"* refers to a diol with a molecular weight from about 62 to 700, and preferably from 62 to 200. These diols may comprise aliphatic, alicyclic or aromatic groups. Preferably, they comprise only aliphatic groups.

Preferably, R₃ represents a low molecular weight diol containing more than 20 carbon atoms, more preferentially chosen from ethylene glycol, diethylene glycol, 1,2-propanediol, 1,3-propanediol, 1,4-butanediol, 1,3-butylene glycol, neopentyl glycol, butylethylpropanediol, cyclohexanediol, 1,4-cyclohexanedimethanol, 1,6-hexanediol, bisphenol A (2,2-bis(4-hydroxyphenyl)propane), hydrogenated bisphenol A (2,2-bis(4-hydroxycyclohexyl)propane), and mixtures thereof.

The low molecular weight diols may optionally comprise ionic or potentially ionic groups. Examples of low molecular weight diols containing ionic or potentially ionic groups are notably described in patent US 3 412 054. Such compounds are preferably chosen from dimethylolbutanoic acid, dimethylolpropionic acid, polycaprolactone diols containing a carboxyl group, and mixtures thereof.

If low molecular weight diols containing ionic or potentially ionic groups are used, they are preferably used in an amount such that less than 0.30 meq of COOH per gram of polyurethane is present in the polyurethane dispersion.

The prepolymer is extended by means of two chain extender families. The first family of chain extenders corresponds to the compounds of general formula (B).

The chain extenders of formula (B) are preferably chosen from alkylenediamines, such as hydrazine, ethylenediamine, propylenediamine, 1,4-butylenediamine, piperazine; alkylenediamine oxides, such as 3-{2-[2-(3-aminopropoxy)ethoxy]ethoxy}propylamine (also known as dipropylamine diethylene glycol or DPA-DEG available from Tomah Products, Milton, Wis.), 2-methyl-1,5-pentanediamine (Dytec A from DuPont), hexanediamine, isophorone diamine, 4,4-methylenedi(cyclohexylamine), ether amines of the DPA series, available from Tomah Products, Milton, Wis., such as dipropylamine propylene glycol, dipropylamine dipropylene glycol, dipropylamine tripropylene glycol, dipropylamine poly(propylene glycol), dipropylamine ethylene glycol, dipropylamine poly(ethylene glycol), dipropylamine 1,3-propanediol, dipropylamine 2-methyl-1,3-propanediol, dipropylamine 1,4-butanediol, dipropylamine 1,3-butanediol, dipropylamine 1,6-hexanediol and dipropylamine cyclohexane-1,4-dimethanol; and mixtures thereof.

The second family of chain extenders corresponds to the compounds of general formula (C). Such compounds preferably have an ionic or potentially ionic group and two groups that can react with isocyanate groups. Such compounds may optionally comprise two groups that react with isocyanate groups and one group which is ionic or capable of forming an ionic group.

The ionic or potentially ionic group may preferably be chosen from ternary or quaternary ammonium groups or groups that can be converted into such groups, a carboxyl group, a carboxylate group, a sulfonic acid group and a sulfonate group. The at least partial conversion of groups that can be converted into a ternary or quaternary ammonium group salt may be performed before or during the mixing with water.

The chain extenders of formula (C) are preferably chosen from diaminosulfonates, for instance the sodium salt of N-(2-aminoethyl)-2-aminoethanesulfonic acid (ASA), the sodium salt of N-(2-aminoethyl)-2-aminopropionic acid, and mixtures thereof.

The polyurethane that may be used according to the present invention may optionally also comprise compounds which are located, respectively, at the chain ends and which terminate said chains (chain terminators). Such compounds are notably described in patents US 7 445 770 and/or US 7 452 770.

Preferably, the aqueous dispersion of polyurethane particles has a viscosity of less than 2000 mPa.s at 23°C, more preferentially less than 1500, and better still less than 1000. Even more preferably, the aqueous polyurethane dispersion has a glass transition temperature of less than 0°C.

Preferably also, the aqueous polyurethane dispersion has a polyurethane (or active material, or solids) content, on the basis of the weight of the dispersion, of from 20% to 60% by weight, more preferentially from 25% to 55% by weight and better still from 30% to 50% by weight. This is intended to mean that the polyurethane content (dry matter) of the aqueous dispersion is preferably from 20% to 60% by weight, more preferentially from 25% to 55% by weight and better still from 30% to 50% by weight, relative to the total weight of the dispersion.

Preferably also, the aqueous dispersion of polyurethane particles has a glass transition temperature (Tg) of less than or equal to -25°C, preferably less than -35°C and more preferentially less than -40°C.

The polyurethane particles may have a mean diameter ranging up to about 1000 nm, for example from about 50 nm to about 800 nm, better still from about 100 nm to about 500 nm. These particle sizes may be measured with a laser particle size analyser (for example Brookhaven BI90).

As non-limiting examples of aqueous polyurethane dispersions, mention may be made of those sold under the name Baycusan^{®} by Bayer, for instance Baycusan^{®} C1000 (INCI name: polyurethane-34), Baycusan^{®} C1001 (INCI name: polyurethane-34), Baycusan^{®} C1003 (INCI name: polyurethane-32), Baycusan^{®} C1004 (INCI name: polyurethane-35) and Baycusan^{®} C1008 (INCI name: polyurethane-48).

Mention may also be made of the aqueous polyurethane dispersions of isophthalic acid/adipic acid copolymer/hexylene glycol/neopentyl glycol/dimethylol acid/isophorone diisocyanate (INCI name: Polyurethane-1, such as Luviset^{®} PUR, BASF), the polyurethane of polycarbonate, polyurethane and aliphatic polyurethane of aliphatic polyester (such as the Neorez^{®} or DSM series, such as Neorez^{®} R989 and Neorez^{®} R-2202).

According to a preferred embodiment, the aqueous dispersion of polyurethane particles may be chosen from aqueous dispersions of particles of compounds having the INCI name polyurethane-35 or compounds having the INCI name polyurethane-34.

Preferably, the compound(s) containing at least one carboxylic function are in the form of aqueous dispersions of particles of acrylic polymers, more preferentially in the form of aqueous dispersions of film-forming acrylic polymer particles.

For the purposes of the invention, the term *"polymer"* means a compound corresponding to the repetition of one or more units (these units being derived from compounds known as monomers). This or these unit(s) are repeated at least twice and preferably at least three times.

The term *"film-forming polymer"* refers to a polymer that is capable of forming, by itself or in the presence of an auxiliary film-forming agent, a macroscopically continuous film on a support, notably on the keratin hair fibers, and preferably a cohesive film.

For the purposes of the present invention, the term *"acrylic polymer"* means a polymer synthesized from at least one monomer chosen from (meth)acrylic acid and/or (meth)acrylic acid ester and/or (meth)acrylic acid amide.

The unit(s) derived from the (meth)acrylic acid monomers of the polymer may optionally be in the form of salt(s), notably of alkali metal, alkaline-earth metal or ammonium salt(s), or organic base salt(s).

The (meth)acrylic acid esters (also known as (meth)acrylates) are advantageously chosen from alkyl (meth)acrylates, in particular C₁ to C₃₀, preferably C₁ to C₂₀ and better still C₁ to C₁₀ alkyl (meth)acrylates, aryl (meth)acrylates, in particular C₆ to C₁₀ aryl (meth)acrylates, and hydroxyalkyl (meth)acrylates, in particular C₂ to C₆ hydroxyalkyl (meth)acrylates.

Among the alkyl (meth)acrylates that may be mentioned are methyl (meth)acrylate, ethyl (meth)acrylate, butyl (meth)acrylate, isobutyl (meth)acrylate, 2-ethylhexyl (meth)acrylate, lauryl (meth)acrylate and cyclohexyl (meth)acrylate.

Among the hydroxyalkyl (meth)acrylates that may be mentioned are hydroxyethyl acrylate, 2-hydroxypropyl acrylate, hydroxyethyl methacrylate and 2-hydroxypropyl methacrylate.

Among the aryl (meth)acrylates that may be mentioned are benzyl acrylate and phenyl acrylate.

The (meth)acrylic acid esters that are particularly preferred are alkyl, preferably C₁ to C₃₀, more preferentially C₁ to C₂₀, better still C₁ to C₁₀, and even more particularly C₁ to C₄, alkyl (meth)acrylates.

According to the present invention, the alkyl group of the esters may be fluorinated, or even perfluorinated, i.e. some or all of the hydrogen atoms of the alkyl group are replaced with fluorine atoms.

As (meth)acrylic acid amides, examples that may be mentioned include (meth)acrylamides and also N-alkyl(meth)acrylamides, in particular N-(C₂ to C₁₂ alkyl)(meth)acrylamides. Among the N-alkyl(meth)acrylamides that may be mentioned are N-ethylacrylamide, N-t-butylacrylamide, N-t-octylacrylamide and N-undecylacrylamide.

The acrylic polymer according to the invention may be a homopolymer or a copolymer, advantageously a copolymer, better still a copolymer of (meth)acrylic acid and of (meth)acrylic acid esters.

Preferably, the acrylic polymer(s) according to the invention comprise one or more units derived from the following monomers:
a) (meth)acrylic acid; and
b) C₁ to C₃₀, more preferentially C₁ to C₂₀, better still C₁ to C₁₀, and even more particularly C₁ to C₄, alkyl (meth)acrylate.

Preferably, the aqueous dispersion of acrylic polymer particles does not comprise any surfactant.

The term "*surfactant*" refers to any agent that is capable of modifying the surface tension between two surfaces.

Among the acrylic polymers according to the invention, mention may be made of copolymers of (meth)acrylic acid and of methyl or ethyl (meth)acrylate, in particular copolymers of methacrylic acid and of ethyl acrylate such as the compound sold under the trade name Luvimer MAE by the company BASF, or the compound Polyacrylate-2 Crosspolymer sold under the trade name Fixate Superhold Polymer by the company Lubrizol, or the compound Acrylate Copolymer sold under the trade name Daitosol 3000VP3 by the company Daito Kasei Kogyo, or the compound Acrylate Polymer sold under the trade name Daitosol 3000 SLPN-PE1 by the company Daito Kasei Kogyo.

The acrylic polymer may optionally comprise one or more additional monomers, other than the (meth)acrylic acid and/or (meth)acrylic acid ester and/or (meth)acrylic acid amide monomers.

As additional monomer, mention will be made, for example, of styrene monomers, in particular styrene and α-methylstyrene, and preferably styrene.

In particular, the acrylic polymer may be a styrene/(meth)acrylate copolymer and notably a polymer chosen from copolymers resulting from the polymerization of at least one styrene monomer and at least one C₁ to C₂₀, preferably C₁ to C₁₀, alkyl (meth)acrylate monomer.

The C₁ to C₁₀ alkyl (meth)acrylate monomer may be chosen from methyl acrylate, ethyl acrylate, propyl acrylate, butyl acrylate, hexyl acrylate, octyl acrylate and 2-ethylhexyl acrylate.

As acrylic polymer, mention may be made of the styrene/(meth)acrylate copolymers sold under the name Joncryl 77 by the company BASF, under the name Yodosol GH41F by the company Akzo Nobel and under the name Syntran 5760 CG by the company Interpolymer.

Preferably, composition C comprises at least one aqueous dispersion of acrylic polymer particles.

More preferentially, composition C comprises at least one aqueous dispersion of acrylic polymer particles comprising one or more units derived from the following monomers:
a) (meth)acrylic acid; and
b) C₁ to C₃₀, more preferentially C₁ to C₂₀, better still C₁ to C₁₀, and even more particularly C₁ to C₄, alkyl (meth)acrylate.

Preferably, the aqueous dispersion of acrylic polymer particles has an acrylic polymer (or active material, or solids) content, on the basis of the weight of the dispersion, of from 20% to 60% by weight, more preferentially from 22% to 55% by weight and better still from 25% to 50% by weight.

### Silicone acrylic copolymer

According to a particular embodiment, the silicone compound(s) comprising at least one carboxylic group are chosen from silicone acrylic copolymers.

Thus, according to a particular embodiment, the compound(s) containing at least one carboxylic function are chosen from silicone acrylic copolymers.

Preferably, said silicone acrylic copolymer(s) comprise:
- at least one acrylic or methacrylic or crotonic unit; and
- at least one polydimethylsiloxane (PDMS) unit.

For the purposes of the present invention, the term "polydimethylsiloxanes" (also abbreviated as PDMSs) denotes, in accordance with what is generally accepted, any organosilicon polymer or oligomer of linear structure, of variable molecular weight, obtained by polymerization and/or polycondensation of suitably functionalized silanes, and constituted essentially of a repetition of main units in which the silicon atoms are linked together via oxygen atoms (siloxane bond Si-O-Si), including methyl radicals directly linked via a carbon atom to said silicon atoms.

The PDMS chains that may be used to obtain the copolymer used according to the invention include at least one polymerizable radical group, preferably located on at least one of the ends of the chain, i.e. the PDMS may contain, for example, a polymerizable radical group on each of the two ends of the chain or a polymerizable radical group on one end of the chain and a trimethylsilyl end group on the other end of the chain.

The term "polymerizable radical group" means a radical that is capable of polymerizing with other polymerizable radical groups or monomers. Preferably, the polydimethylsiloxane unit comprises at least one polymerizable radical group.

Preferably, the polymerizable radical group comprises at least one vinyl group.

Preferably, the polydimethylsiloxane (PDMS) unit comprises at least one polymerizable radical group comprising at least one vinyl group, preferably at least two polymerizable radical groups comprising at least one vinyl group, preferably located on at least one of the chain ends.

As indicated previously, said silicone acrylic copolymer(s) preferably comprise(s) at least one acrylic or methacrylic or crotonic unit, i.e. at least one unit comprising a carboxylic group.

The term "carboxylic group" means a COOH or COO⁻ functional group, the counterion of the COO⁻ group possibly being chosen from alkali metals, alkaline-earth metals and quaternary ammoniums.

Preferably, said silicone acrylic copolymer(s) comprise:
- at least one acrylic or methacrylic or crotonic unit, and at least one acrylic ester or methacrylic ester or vinyl ester unit; and
- at least one polydimethylsiloxane (PDMS) unit.

More preferentially, the composition comprises one or more silicone acrylic copolymers comprising:
- at least one crotonic unit and at least one unit chosen from an alkyl crotonate unit, the alkyl radical being a linear or branched, saturated radical containing from 1 to 20 carbon atoms; a vinyl acetate unit; a vinyl alkyl ester unit, the alkyl radical being a linear or branched, saturated radical containing from 2 to 20 carbon atoms; and mixtures thereof; and
- at least one polydimethylsiloxane (PDMS) unit.

The term "crotonic unit" means a unit derived from a crotonic acid monomer or a salt thereof.

The term "alkyl crotonate unit" means a unit derived from a crotonic acid ester monomer containing a saturated, linear or branched alkyl radical containing from 1 to 20 carbon atoms.

The term "vinyl alkyl ester unit" means a unit derived from a vinyl ester monomer containing a saturated, linear or branched alkyl radical containing from 2 to 20 carbon atoms.

The term "vinyl acetate unit" means a unit derived from a vinyl acetate monomer.

According to a preferred embodiment, said silicone acrylic copolymer(s) comprise:
- at least one crotonic unit, at least one vinyl acetate unit and at least one vinyl alkyl ester unit, the alkyl radical being a saturated, linear or branched radical containing from 2 to 20 carbon atoms, preferably from 2 to 18 carbon atoms, and
- at least one polydimethylsiloxane (PDMS) unit preferably including at least one polymerizable radical group comprising at least one vinyl group.

According to a particularly preferred embodiment, said silicone acrylic copolymer(s) comprise:
- at least one crotonic unit, at least one vinyl acetate unit and at least one vinyl alkyl ester unit, the alkyl radical being a linear or branched, saturated radical containing from 6 to 16 carbon atoms, and

- at least one polydimethylsiloxane (PDMS) unit comprising at least one polymerizable radical group comprising at least one vinyl group.

More preferentially, the composition comprises one or more silicone acrylic copolymers comprising:
- at least one crotonic unit, at least one vinyl acetate unit and at least one vinyl alkyl ester unit, the alkyl radical being a saturated, linear or branched radical containing from 2 to 20 carbon atoms, preferably from 2 to 18 carbon atoms; and
- at least one polydimethylsiloxane (PDMS) unit including at least one polymerizable radical group comprising at least one vinyl group.

Even more preferentially, the composition comprises one or more silicone acrylic copolymers comprising:
- at least one crotonic unit, at least one vinyl acetate unit and at least one vinyl alkyl ester unit, the alkyl radical being a saturated, linear or branched radical containing from 6 to 16 carbon atoms; and
- at least one polydimethylsiloxane (PDMS) unit including at least one polymerizable radical group comprising at least one vinyl group.

Among the silicone acrylic copolymers that may be used in the context according to the invention, mention may be made of the compound sold by the company Wacker Chemie AG under the trade name Belsil^{®} P1101, having the INCI name Crotonic Acid/Vinyl C8-12 Isoalkyl Esters/VA/Bis-Vinyldimethicone Crosspolymer.

The total amount of the compound(s) containing at least one carboxylic function preferably ranges from 0.1% to 30% by weight, more preferentially from 0.5% to 20% by weight, better still from 1% to 15% by weight, and even more preferentially from 2% to 10% by weight, relative to the total weight of composition C.

The total amount of the aqueous dispersion(s) of particles of polymer(s) chosen from polyurethanes, acrylic polymers, and mixtures thereof preferably ranges from 0.1% to 40% by weight, more preferentially from 0.5% to 30% by weight, better still from 1% to 25% by weight, and even more preferentially from 2% to 20% by weight, relative to the total weight of composition C.

According to a particular embodiment, the total amount of the aqueous dispersion(s) of acrylic polymer particle(s) preferably ranges from 0.1% to 40% by weight, more preferentially from 0.5% to 30% by weight, better still from 1% to 25% by weight, and even more preferentially from 2% to 20% by weight, relative to the total weight of composition C.

Advantageously, the total amount of silicone acrylic copolymer(s) ranges from 0.1% to 30% by weight, more preferentially from 0.5% to 20% by weight, better still from 1% to 15% by weight and even more preferentially from 2% to 10% by weight relative to the total weight of composition C.

### Organic solvents

Composition C according to the invention may comprise one or more organic solvents.

Examples of organic solvents that may be mentioned include C₁-C₄ lower alkanols, such as ethanol and isopropanol; polyols and polyol ethers, for instance 2-butoxyethanol, 1,2-hexanediol, propylene glycol, pentylene glycol, propylene glycol monomethyl ether, diethylene glycol monomethyl ether and monoethyl ether, and also aromatic alcohols, notably aromatic monoalcohols, for instance benzyl alcohol or phenoxyethanol, and mixtures thereof.

The organic solvents may be present in a total amount of between 0.01% and 75% by weight, preferably between 0.05% and 50% by weight and more preferentially inclusively between 0.1% and 45% by weight relative to the total weight of composition C.

Composition C used in the context of the process according to the invention may be aqueous. The water content may range from 1% to 90% by weight, preferably from 10% to 80% by weight and more preferentially from 20% to 75% by weight relative to the total weight of composition C.

### Additives

Composition C used in the context of the process according to the invention may contain any adjuvant or additive usually used.

Among the additives that may be contained in the composition, mention may be made of reducing agents, softeners, antifoams, moisturizers, UV-screening agents, peptizers, fragrances, anionic, cationic, nonionic or amphoteric surfactants, proteins, vitamins, thickeners, preserving agents, silicones, oils, waxes other than silicones in wax form, and mixtures thereof.

Composition C used in the context of the process according to the invention may notably be in the form of a suspension, a dispersion, a gel, an emulsion, notably an oil-in-water (O/W) or water-in-oil (W/O) emulsion, or a multiple emulsion (W/O/W or polyol/O/W or O/W/O), in the form of a cream, a mousse, a stick, a dispersion of vesicles, notably of ionic or nonionic lipids, or a two-phase or multi-phase lotion.

A person skilled in the art may select the appropriate presentation form, and also the method for preparing it, on the basis of his general knowledge, taking into account firstly the nature of the constituents used, notably their solubility in the support, and secondly the intended application of the composition.

### Airbrush

As indicated previously, composition C used in the context of the process according to the invention is sprayed onto the keratin hair fibers using an airbrush.

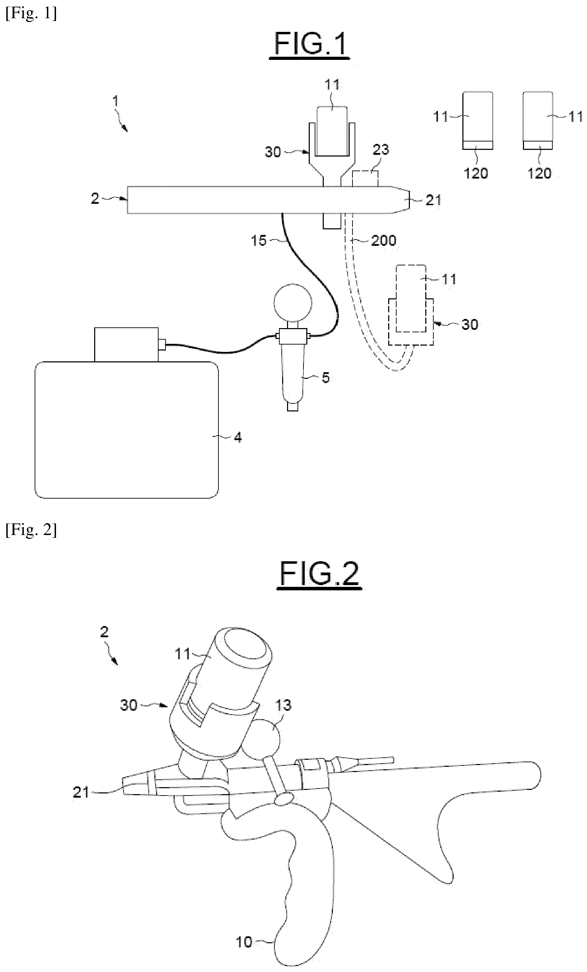

To spray the composition just described, an airbrush system 1 as shown in Figure 1 is used.

The airbrush system 1 may include, as illustrated, an airbrush 2 connected to a source of compressed gas, for example including an air compressor 4 connected to the airbrush 2 via a pressure regulator 5, in a manner known *per se,* and a hose 15. The source of compressed gas may also be an interchangeable or refillable capsule of compressed gas, for example of compressed air.

The airbrush 2 includes, in a manner known *per se,* a gripping part which is defined, for example, by the elongated body of the airbrush when the latter is penshaped or by a handle 10 when it is pistol-shaped, as illustrated in Figure 2.

The airbrush 2 may carry a container 11 containing the composition C to be sprayed, this container 11 being, for example, in the form of a removable bottle. The airbrush may include, as illustrated, at the top, a connecting device 30 for receiving the container 11.

During operation of the airbrush 2, the product is sucked up and flows into the airbrush via a collection channel before being sprayed.

The container 11 may be transparent or provided with graduations so as to allow the user to more readily visualize the amount of product available. The volume of product contained in the container 11 is, for example, between 1 and 5000 mL and preferably 1 to 1000 mL, even more preferentially 1 to 500 mL.

Preferably, the hose 15 which connects the airbrush to the compressor 4 and notably to the air regulator 5 is less than or equal to 5 m long and its inside diameter is equal to 4 mm, for example. The hose 15 is preferably fitted with quick-connect couplings.

The airbrush 2 may be proposed to the user with several stoppered and prefilled containers 11, as illustrated in Figure 1, for example all containing the same product so as to allow the user to quickly replace an empty container with a full one, for example to treat different people.

The containers 11 may even include different products, for example products of different compositions, intended to perform different treatments, complementary or otherwise, and the user can choose which of the containers 11 corresponds to the treatment to be performed.

The spray parameters, notably the flow rate of the carrier gas (preferably air) and/or the flow rate of the sprayed product, may be adapted manually by the user each time the container 11 is changed, when necessary, or during use to obtain the desired visual effect.

The relative pressure of the compressed air entering the airbrush 2 may be between 0.2 and 3 bar, preferably between 0.4 and 1 bar, for example being about 0.5 bar.

The nozzle 21 fitted to the airbrush is preferably chosen so that the average size of the droplets of sprayed product is centred on a value of between 10 and 35 µm, for example being about 23 µm (size measured at a distance of 15 cm from the nozzle outlet).

The nozzle 21 may have an outlet diameter ranging, for example, from 0.1 to 1 mm, preferably from 0.2 to 0.7 mm.

The connecting device 30, suitable for a container 11 in the form of a bottle, including a neck fitted with a collar and a stopper 120, includes, in the example illustrated, an end piece for fixing to the body of the airbrush, for example of the quickfit type, so as to allow removable fixing to the body of the airbrush.

Needless to say, it would not be a departure from the context of the present invention if the connecting device 30 were not removably fixed to the rest of the airbrush or were removably fixed using means other than a quick coupling.

When the connecting device 30 is in place on the airbrush, the axis of the nozzle may be vertical or oriented obliquely towards the front or rear of the airbrush.

The connecting device 30 may even be made in the form of a part of the body of the airbrush, the connecting device 30 not necessarily projecting relative to the body of the airbrush.

Since the container 11 is fixed in a leaktight manner to the connecting device, an air return circuit is provided to allow air to flow back into the container as it is emptied. The container is closed, except for the product collection and air return circuits.

To use the airbrush 2, the user places the container 11 on the connecting device 30.

Before being placed on the airbrush, the container 11 is for example fitted with a stopper 120 such as a nipple or a closing cap, which is then separated from the container.

The airbrush 2 may include any spray triggering means, for example in the form of a control member such as a lever 13, as illustrated in Figure 2, actuated by a finger of the hand of the user holding the airbrush. In this figure, the airbrush is a gravity airbrush. It is not outside the scope of the invention to use a suction airbrush. In the latter case, it is sufficient for the connecting device 30 to be fitted with a system (for example a tube 200 as shown in the dashed line in Figure 1) for connecting the first orifice to the inlet of the airbrush, allowing suction.

As examples of airbrushes to which the invention may be applied, mention may be made of the reference A 4700 from the company Azteck, the reference Kustom micron CM from the company Iwata, and the reference "Evolution Infinity 2 in 1" from the company Harder and Steenbeck, these airbrushes being of the pen grip type. Use may also be made of the "Kustom revolution TR" or "Studio series" airbrushes from the company Iwata, or the Colani^{®} airbrush from the company Harder & Steenbeck.

Needless to say, arrangements other than those just described are possible. For example, the same compressor may be used for several airbrushes, this compressor being located, for example, outside the room in which the treatment is performed, so as to reduce noise pollution.

Preferably, the spray is circular, but various nozzles can be used, so as to have flat sprays or sprays having another shape depending on the visual effect to be obtained.

The compressor may be replaced with a supply of compressed air, for example bottled compressed air or a compressed or liquefied gas cartridge, for example carried on the airbrush and handled with it during use.

Several containers may be mounted on the airbrush, where appropriate.

A tank carried by the airbrush may be replaced with a tank connected to the airbrush by a pipe, the product being sucked into the airbrush by the negative pressure created by the Venturi effect by the circulation of carrier gas in the airbrush. A pump may also be used to feed the product to be sprayed to the airbrush.

The use of an airbrush allows the operating parameters to be easily modified to change the effects generated, during use, at the user's discretion; said user can, for example, adjust the flow rate of the jet of product as a function of the desired visual effect. Furthermore, the volume of the gas (preferably air) used as a vector is unlimited when the airbrush is connected to a compressor. The product to be sprayed can be readily renewed and different products can easily be used successively with the same airbrush.

The flow rate of the sprayed composition is advantageously less than or equal to 10 g/min.

### Protocol

Composition C described above may be used on wet or dry keratin hair fibers, and also on any type of fair or dark, natural or dyed, permanent-waved, bleached or relaxed keratin hair fibers.

According to a particular embodiment of the invention, the keratin hair fibers is washed before applying composition C.

Preferably, a washing, rinsing, draining or drying step is performed after the application of composition C to the keratin hair fibers.

More preferentially, a drying step is performed after spraying composition C onto the keratin hair fibers.

As indicated previously, composition C is sprayed using an airbrush as described above.

Composition C is generally sprayed on the keratin hair fibers at room temperature (between 15 and 25°C).

After spraying composition C on the keratin hair fibers, it is possible to wait for between 1 minute and 6 hours, in particular between 1 minute and 2 hours, more particularly between 1 minute and 1 hour, more preferentially between 1 minute and 30 minutes, before, for example, a washing, rinsing, draining and/or drying step.

After spraying composition C, the keratin hair fibers may be left to dry or may be dried, for example at a temperature of greater than or equal to 30°C.

The process according to the invention may thus comprise a step of applying heat to the keratin hair fibers using a heating tool.

The heat application step of the process of the invention may be performed using a hood, a hair dryer, a straightening iron, a curling iron, a Climazon hood, etc.

Preferably, the heat application step of the process of the invention is performed using a hair dryer.

When the process of the invention involves a step of applying heat to the keratin hair fibers, the step of applying heat to the keratin hair fibers takes place after applying composition C to the keratin hair fibers.

During the step of applying heat to the keratin hair fibers, a mechanical action may be exerted on the locks, such as combing, brushing or running the fingers through.

When the step of applying heat to the keratin hair fibers is performed using a hood or a hair dryer, the temperature is preferably between 30°C and 110°C, preferentially between 50°C and 90°C.

When the step of applying heat to the keratin hair fibers is performed using a straightening iron, the temperature is preferably between 110°C and 220°C, preferably between 140°C and 200°C.

In a particular variant, the process of the invention involves a step (c1) of applying heat using a hood, a hair dryer or a Climazon hood, preferably a hair dryer, and a step (c2) of applying heat using a straightening or curling iron, preferably a straightening iron.

Step (c1) may be performed before step (c2).

During step (c1), also referred to as the drying step, the keratin hair fibers may be dried, for example at a temperature above or equal to 30°C. According to a particular embodiment, this temperature is above 40°C. According to a particular embodiment, this temperature is above 45°C and below 110°C.

Preferably, if the keratin hair fibers is dried, it is dried, in addition to a supply of heat, with a flow of air. This flow of air during drying makes it possible to improve the strand separation of the coating.

During the drying, a mechanical action may be exerted on the locks, such as combing, brushing or running the fingers through.

During step (c2), the passage of the straightening or curling iron, preferably the straightening iron, may be performed at a temperature ranging from 110°C to 220°C, preferably between 140°C and 200°C.

After the heating step, a shaping step may be performed, for example with a straightening iron; the temperature for the shaping step may be between 110 and 220°C, preferably between 140 and 200°C.

Preferably, the invention is a process for dyeing keratin hair fibers comprising the following steps:
i) spraying the keratin hair fibers, using an airbrush as described above, with at least one composition C comprising:
   - at least one (poly)carbodiimide compound as described previously; and
   - at least one colouring agent chosen from pigments, direct dyes and mixtures thereof; and then
ii) optionally a leave-on time of said composition C on the keratin hair fibers of from 1 minute to 30 minutes, preferably from 1 to 20 minutes; and then
iii) optionally a step of washing, rinsing, draining and/or drying said keratin hair fibers.

Preferably, composition C also comprises at least one compound containing at least one carboxylic function.

Advantageously, the spraying of composition C on the keratin hair fibers is repeated several times.

According to a preferred embodiment, the dyeing process according to the invention is a process for dyeing keratin hair fibers which consists in extemporaneously mixing, at the time of use, at least two compositions A and B to obtain a composition C and in spraying composition C on the keratin hair fibers, using an airbrush as described above, with:
- composition A comprising at least one (poly)carbodiimide compound as described previously;
- composition B comprising at least one colouring agent chosen from pigments, direct dyes and mixtures thereof.

Composition A and/or composition B may optionally comprise at least one compound containing at least one carboxylic function.

Preferably, composition B comprises at least one compound containing at least one carboxylic function.

Preferably, composition A does not comprise at least one colouring agent chosen from pigments, direct dyes and mixtures thereof.

According to this embodiment, compositions A and B are mixed preferably less than 15 minutes before spraying on the keratin hair fibers, more preferentially less than 10 minutes before spraying, better still less than 5 minutes before spraying.

The weight ratio between composition A and composition B preferably ranges from 0.1 to 10, preferentially from 0.2 to 5 and better still from 0.5 to 2, or even from 0.6 to 1.5. In a particular embodiment, the weight ratio between composition A and composition B is equal to 1.

The total amount of the (poly)carbodiimide compound(s) preferably ranges from 0.01% to 40% by weight, more preferentially from 0.1% to 30% by weight, better still from 0.5% to 20% by weight and even more preferentially from 1% to 12% by weight relative to the total weight of composition A.

When it is (they are) present, the total amount of the compound(s) containing at least one carboxylic function preferably ranges from 0.2% to 60% by weight, more preferentially from 1% to 40% by weight, better still from 1% to 30% by weight, and even more preferentially from 2% to 20% by weight, relative to the total weight of composition B.

### Spraying system

The present invention also relates to a spraying system comprising:
- an airbrush;
- at least one compartment containing a composition C comprising:
   a) at least one (poly)carbodiimide compound as defined above;
   b) at least one colouring agent chosen from pigments, direct dyes and mixtures thereof.

The present invention also relates to a spraying system comprising:
- an airbrush;
- several compartments containing:
   in a first compartment, a composition A comprising:
      a) at least one (poly)carbodiimide compound as defined above;
   in a second compartment, a composition B comprising:
      b) at least one colouring agent chosen from pigments, direct dyes and mixtures thereof.

The present invention will now be described more specifically by means of examples, which do not in any way limit the scope of the invention. However, the examples make it possible to support specific features, variants and preferred embodiments of the invention.

### EXAMPLES

The (poly)carbodiimide(s) of the invention are accessible via synthetic methods known to those skilled in the art starting from commercial products or reagents that can be synthesized according to chemical reactions that are also known to those skilled in the art. Mention may be made, for example, of the book Sciences of Synthesis - Houben - Weyl Methods of Molecular Transformations, 2005, Georg Thiem Verlag Kg, Rudigerstrasse 14, D-70469 Stuttgart, or the American patent US 4 284 730 or the Canadian patent application CA 2 509 861.

More particularly, the process for preparing the (poly)carbodiimides of the invention involves, in a first step, a diisocyanate reagent (1):

O=C=N-L₁-N=C=O (1),

in which formula (1) L₁ is as defined previously, which reacts in the presence of a carboimidation catalyst (2) such as those described in US 4 284 730, notably phosphorus-based catalysts particularly chosen from phospholene oxides and phospholene sulfoxides, diaza- and oxaza-phospholanes, preferably under an inert atmosphere (nitrogen or argon), and in particular in a polar solvent which is preferably aprotic such as THF, glyme, diglyme, 1,4-dioxane or DMF, at a temperature between room temperature and the reflux temperature of the solvent, preferably at about 140°C; to give the carbodiimide diisocyanate compound (3):

O=C=N-L₁-(N=C=N-L₁)ₙ-N=C=O (3),

in which formula (3) L₁ and n are as defined previously. Benzoyl halogen such as benzoyl chloride may be added to deactivate the catalyst.

To obtain "symmetrical" (poly)carbodiimides, during the second step of the preparation process, compound (3) reacts with 1 molar equivalent (1 eq.) of nucleophilic reagent R₁-X₁-H and then 0.5 eq. of reagent H-E-H with R₁, X₁ and E as defined previously, to give the "symmetrical" compound (4) according to the invention:

[R₁-X₁-C(O)-NH-L₁-(N=C=N-L₁)ₙ-NH-C(O)]₂-E (4),

in which formula (4) R₁, X₁, L₁, n and E are as defined previously. According to one variant to obtain compound (4) from (3), it is possible first to add 0.5 eq. of reagent H-E-H and then 1 eq. of reagent R₁-X₁-H.

To obtain "dissymmetrical" (poly)carbodiimides, during the second step of the preparation process, compound (3) reacts with 1 molar equivalent (1 eq.) of nucleophilic reagent R₁-X₁-H and then 1 eq. of reagent H-E-H with R₁, X₁ and E as defined previously, to give compound (5):

R₁-X₁-C(O)-NH-L₁-(N=C=N-L₁)ₙ-NH-C(O)-E-H (5),

in which formula (5) R₁, X₁, L₁, n and E are as defined previously.

According to one variant to obtain compound (5) from (3), it is possible first to add 1 eq. of reagent R₁-X₁-H and then 0.5 eq. of reagent H-E-H.

During a third step, compound (5) reacts with 1 eq. of compound (6)

R₂-X₂-C(O)-NH-L₁-(N=C=N-L₁)_{z}-N=C=O (6), said compound (6) is prepared beforehand from compound (3'):

O=C=N-L₁-(N=C=N-L₁)_{z}-N=C=O (3'),

in which formula (3') L₁ and z are as defined previously, which reacts with 1 eq. of nucleophilic reagent R₂-X₂-H with L₁, R₂, X₂ and z as defined previously, to give the dissymmetrical compound (7):

R₁-X₁-C(O)-NH-L₁-(N=C=N-L₁)ₙ-NH-C(O)-E-C(O)-NH-L₁-(N=C=N-L₁)_{z}-NH-C(O)-X₂-R₂ (7),

in which formula (7) R₁, X₁, L₁, R₂, X₂, n, z and E are as defined previously.

It is also possible to react 1 molar equivalent of compound O=C=N-L₁-(N=C=N-L₁)_{z}-N=C=O (3') with 1/w molar equivalent of H-E-H, followed by 1 eq. of nucleophilic reagent R₂-X₂-H to give compound (8):

H-[E-C(O)-NH-L₁-(N=C=N-L₁)_{z}]_{w}-NH-C(O)-X₂-R₂ (8),

in which formula (8) L₁, R₂, X₂, z and E are as defined previously, and w is an integer between 1 and 3; more preferentially, w = 1.

This last compound (8) can then react with 1 eq. of compound (4'):

R₁-X₁-C(O)-NH-L₁-(N=C=N-L₁)ₙ-N=C=O (4'),

(said compound (4') being able to be synthesized by reaction of 0.5 eq. of nucleophilic reagent R₁-X₁-H with 1 equivalent of compound (3)), to give the (poly)carbodiimide (9) of the invention:

R₁-X₁-C(O)-NH-L₁-(N=C=N-L₁)ₙ-NH-C(O)-[E-C(O)-NH-L₁-(N=C=N-L₁)_{z}]_{w}-NH C(O)-X₂-R₂ (9),

in which formula (9) L₁, R₁, X₁, R₂, X₂, n, z, w and E are as defined previously.

The (poly)carbodiimide compounds, and similarly all the reaction intermediates and reagents, may be purified via conventional methods known to those skilled in the art, such as extraction with water and water-immiscible organic solvent, precipitation, centrifugation, filtration and/or chromatography.

### Example 1: Process for synthesizing the (poly)carbodiimide compound

50 g of 4,4'-dicyclohexylmethane diisocyanate and 0.5 g of 4,5-dihydro-3-methyl-1-phenyl-1H-phosphole 1-oxide were placed with stirring in a 500 mL threenecked round-bottomed flask equipped with a thermometer, a stirrer and a reflux tube.

The reaction medium was heated at 140°C under nitrogen for 4 hours, the reaction being monitored by infrared spectroscopy by means of the absorption of the isocyanate functions between 2200 and 2300 cm⁻¹, and then cooled to 120°C.

A mixture of 5.3 g of polyethylene glycol monomethyl ether and 1.2 g of 1,4-butanediol are introduced with stirring into the reaction medium. The temperature of 120°C is maintained until the isocyanate functions have totally disappeared, monitored by infrared spectroscopy at 2200-2300 cm⁻¹, and is then cooled to room temperature.

After cooling to room temperature, the reaction medium is poured dropwise with vigorous stirring into a 500 mL glass beaker containing 85 g of distilled water, to give the desired product in the form of a translucent yellow liquid.

### Example 2

Compositions A and B as described in Tables 1 and 2 below were prepared: the amounts are expressed as g of starting material as obtained/100 g, unless otherwise mentioned. In the table below, "a.m." means "active material".

**[Table 1]**

| **Composition** | **A** |
|---|---|
| Polycarbodiimide ⁽¹⁾ | 7 a.m. |
| Ethanol | 20 |
| Water | qs 100 |

(1) synthesized according to the synthetic process described in Example 1 (containing 40% active material in water).

**[Table 2]**

| **Composition** | **B** |
|---|---|
| Acrylates copolymer ⁽²⁾ | 29.2 |
| Yellow iron oxide | 2.3 |
| Nacre (mica (and) iron oxides) | 9.7 |
| Bis-hydroxy/methoxy/methyl amodimethicone crosspolymer (and) Trideceth-10 | 8.8 |
| Divinyl dimethicone/dimethicone (and) C₁₂-₁₃ Pareth-3 (and) C₁₂-₁₃ Pareth-23 copolymer⁽³⁾ | 10.2 |
| Ethanol | 3.75 |
| Preserving agent(s) | qs |
| Water | qs 100 |

(2) sold by the company Daito Kasei Kogyo under the trade name Daitosol 3000SLPN-PE1 (aqueous dispersion containing 30% active material)
(3) sold by the company Dow Corning under the name Dow Corning HMW 2220 Nonionic Emulsion (divinyl dimethicone/dimethicone block copolymer as an aqueous emulsion) (60% active material)

Composition A was then mixed with composition B in a 50/50 mass ratio to obtain a composition C according to the invention.

### Protocols

Two application processes were used.

According to the first process, known as process P1, composition C was applied manually using a brush onto locks of dry natural keratin hair fibers containing 90% white keratin hair fibers strands, at a rate of 0.8 g of composition per gram of lock. The locks of keratin hair fibers were then disentangled and dried with a hair dryer, and then combed.

This is thus a comparative process.

According to the second process, known as process P2, composition C was introduced into a cup, which was inserted into the airbrush (Iwata Studio series airbrush). Pressure was exerted on the trigger to distribute the composition via compressed air at 0.5 bar at a distance of no more than 5 cm, for a time sufficient to cover the lock of dry natural keratin hair fibers containing 90% white keratin hair fibers strands. Composition C was thus sprayed onto the locks of keratin hair fibers. The locks of keratin hair fibers were then disentangled and dried with a hair dryer, and then combed.

This is thus a process according to the invention.

### Shampoo washing protocol:

The locks of dyed keratin hair fibers are combed, moistened with water at 35°C and then passed between the fingers five times for 5 seconds. The locks of keratin hair fibers are then squeezed dry between two fingers.

A standard shampoo (Garnier Ultra Doux) is applied uniformly to the dyed locks, in a proportion of 0.4g of standard shampoo per gram of locks, the locks of keratin hair fibers being massaged gently along the length (6 passes) for 15 seconds, from the root to the end.

The locks of keratin hair fibers are then placed on a watch glass and left to stand for 1 minute.

Next, the locks of keratin hair fibers are rinsed with water while passing the locks between the fingers (15 passes). The locks of keratin hair fibers are then squeezed dry between two fingers before the next shampoo wash.

Once the tests of several shampoo washes have been performed, the locks of keratin hair fibers are combed and dried with a hair dryer.

### Results

The persistence of the colour of the locks was evaluated in the CIE L*a*b* system, using a Minolta Spectrophotometer CM3600A colorimeter (illuminant D65, angle 10°, specular component included).

In this L*a*b* system, L* represents the intensity of the colour, a* indicates the green/red colour axis and b* the blue/yellow colour axis.

The persistence of the colouring is evaluated by the colour difference ΔE between the coloured locks before shampoo washing, then after having undergone three shampoo washes according to the protocol described above. The lower the ΔE value, the more persistent the colour with respect to shampoo washing.

The ΔE value is calculated according to the following equation: $ΔE = \sqrt{{\left({L}^{*}-{{L}_{o}}^{*}\right)}^{2} + {\left({a}^{*}-{{a}_{o}}^{*}\right)}^{2} + {\left({b}^{*}-{{b}_{o}}^{*}\right)}^{2}}$

In this equation, L*a*b* represent the values measured after dyeing the keratin hair fibers and after performing the shampoo washes, and L₀*a₀*b₀* represent the values measured after dyeing the keratin hair fibers but before shampoo washing.

**[Table 3]**

| Processes | Number of shampoo washes | L* | a* | b* | ΔE |
|---|---|---|---|---|---|
| P1 (comparative) | 0 | 54.4 | 21.0 | 31.8 | - |
| | 3 | 67.6 | 5.9 | 20.3 | 23.1 |
| P2 (invention) | 0 | 54.2 | 21.0 | 31.0 | - |
| | 3 | 58.9 | 16.7 | 26.5 | 7.8 |

The locks of keratin hair fibers treated by means of process P2 according to the invention and washed with three shampoo washes have a lower ΔE value than that of the locks of keratin hair fibers treated by means of the comparative process P1.

Thus, the coloured coating that is obtained by means of the processes according to the invention shows improved persistence with respect to shampoo washing.

## Claims

1. Process for dyeing keratin hair fibers which involves spraying the keratin hair fibers with at least one composition C comprising:
- one or more (poly)carbodiimide compounds; and
- at least one colouring agent chosen from pigments, direct dyes and mixtures thereof, using an airbrush.

2. Process according to Claim 1, **characterized in that** the (poly)carbodiimide compound(s) are chosen from the compounds of formula (I) below: 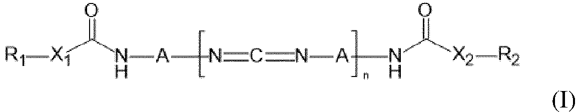 in which:
- X₁ and X₂ independently represent an oxygen atom O, a sulfur atom S or an NH group;
- R₁ and R₂ independently represent a group chosen from a hydrocarbon-based radical, preferably alkyl, optionally interrupted with one or more heteroatoms, a group chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups, and a hydrocarbon-based radical, preferably alkyl, optionally interrupted with one or more heteroatoms and with one or more groups chosen from alkoxysilyl, hydroxysilyl, acetoxysilyl, vinylsilyl, acrylalkylsilyl, methacrylalkylsilyl, crotonylalkylsilyl, carboxyanhydridoalkylsilyl, carboxyalkylsilyl, hydroxyalkylsilyl, aldehydoalkylsilyl, mercaptoalkylsilyl, norbornenylsilyl, acylpentadienylalkylsilyl, maleimidoalkylsilyl, sulfonylalkylsilyl, (meth)acrylalkyl, crotonylalkyl, alkylepoxide such as propylepoxide or butylepoxide and azacyclopropane groups;
- n denotes an integer ranging from 1 to 1000; and
- A is a monomer chosen from the compounds below:

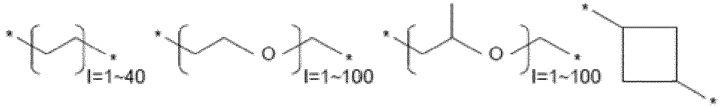

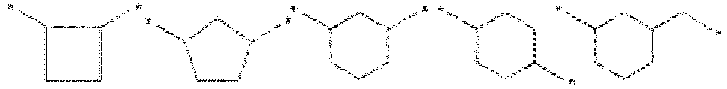

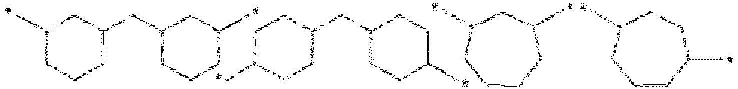

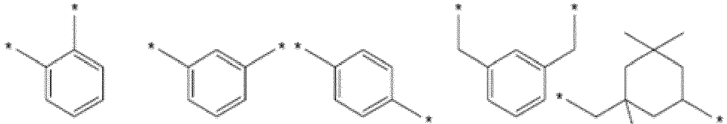

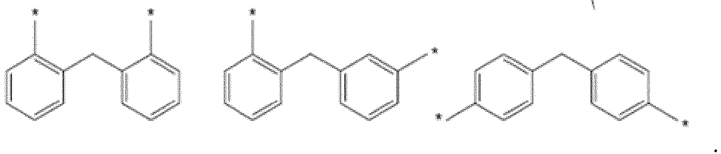

3. Process according to Claim 1, **characterized in that** the (poly)carbodiimide compound(s) are chosen from the compounds of formula (II) below: 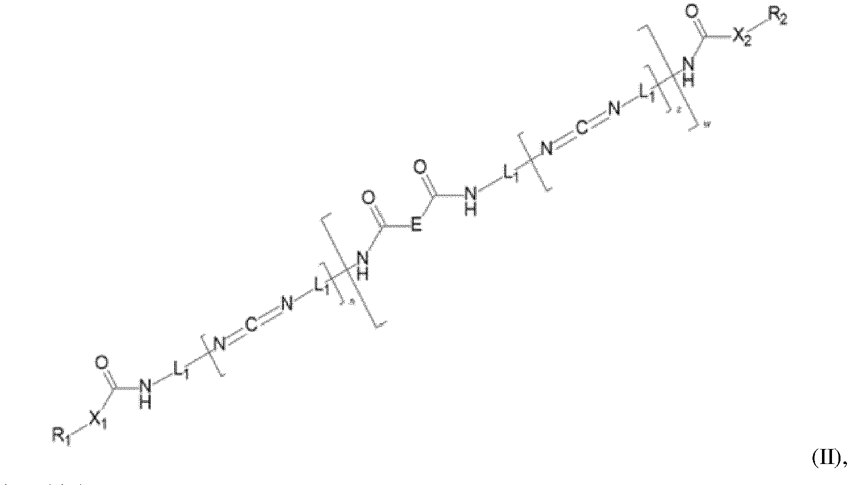 in which:
- X₁ and X₂ independently represent an oxygen atom O, a sulfur atom S or an NH group;
- R₁ and R₂ independently represent a hydrocarbon-based radical optionally interrupted with one or more heteroatoms;
- n and z denote an integer ranging from 1 to 20, with n+z ≥ 2 and w denotes an integer ranging from 1 to 3;
- L₁ independently represents a C₁-C₁₈ divalent aliphatic hydrocarbon-based radical, a C₃-C₁₅ cycloalkylene radical, a C₃-C₁₂ heterocycloalkylene group or a C₆-C₁₄ arylene group, and mixtures thereof;
- E independently represents a group chosen from:
-O-R₃-O-; -S-R₄-S-; -R₅-N(R₆)-R₄-N(R₆)-R₅-;
in which R₃ and R₄ independently represent a divalent hydrocarbon-based radical optionally interrupted with one or more heteroatoms;
- R₅ independently represents a covalent bond or a saturated divalent hydrocarbon-based radical, optionally interrupted with one or more heteroatoms;
- R₆ independently represents a hydrogen atom or a hydrocarbon-based radical, optionally interrupted with one or more heteroatoms.

4. Process according to Claim 3, **characterized in that** the (poly)carbodiimide compound(s) are chosen from the compounds of formula (II) in which:
- X₁ and X₂ independently represent an oxygen atom;
- R₁ and R₂ are independently chosen from dialkylamino alcohols, alkyl esters of hydroxycarboxylic acid and monoalkyl ethers of (poly)alkylene glycol, in which a hydroxyl group has been removed, and mixtures thereof;
- n and z denote an integer ranging from 1 to 20, with n+z ≥ 2 and w is equal to 1;
- L1 is chosen from a C₁-C₁₈ divalent aliphatic hydrocarbon-based radical, a C₃-C₁₅ cycloalkylene radical, a C₃-C₁₂ heterocycloalkylene group or a C₆-C₁₄ arylene group, and mixtures thereof;
- E independently represents a group chosen from:
-O-R₃-O-; -S-R₄-S-; -R₅-N(R₆)-R₄-N(R₆)-R₅-;
in which R3 and R4 are independently chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof;
- when R₅ is not a covalent bond, R₅ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof; and
- R₆ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof.

5. Process according to Claim 3 or 4, **characterized in that** the (poly)carbodiimide compound(s) are chosen from the compounds of formula (II) in which:
- X₁ and X₂ independently represent an oxygen atom;
- R₁ and R₂ are, independently, monoalkyl ethers of (poly)alkylene glycol, in which a hydroxyl group has been removed;
- n and z denote an integer ranging from 1 to 20, with n+z ≥ 2 and w is equal to 1;
- L₁ is a C₃-C₁₅ cycloalkylene radical;
- E independently represents a group chosen from:
-O-R₃-O-; -S-R₄-S-; -R₅-N(R₆)-R₄-N(R₆)-R₅-;
in which R₃ and R₄ are independently chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof;
- when R₅ is not a covalent bond, R₅ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof; and
- R₆ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof.

6. Process according to any one of Claims 3 to 5, **characterized in that** the (poly)carbodiimide compound(s) are chosen from the compounds of formula (II) in which:
- X₁ and X₂ independently represent an oxygen atom;
- R₁ and R₂ independently represent the compound of formula (VI) below:
R₁₃-[O-CH₂-C(H)(R₁₄)]_{q}- (VI),
in which R₁₃ represents a C₁-C₄ alkyl group or a phenyl, preferably a C₁-C₄ alkyl group, more preferentially a methyl, R₁₄ represents a hydrogen atom or a C₁-C₄ alkyl group, preferably a hydrogen atom and q denotes an integer ranging from 4 to 30;
- n and z denote an integer ranging from 1 to 20, with n+z ranging from 4 to 10 and w is equal to 1;
- L₁ is a C₃-C₁₅ cycloalkylene radical such as cyclopentylene, cycloheptylene, cyclohexylene and 4,4-dicyclohexylenemethane; and
- E represents a group -O-R₃-O- in which R₃ is chosen from a C₆-C₁₄ arylene radical, a C₃-C₁₂ cycloalkylene radical, a linear or branched C₁-C₁₈ alkylene radical, optionally interrupted with one or more heteroatoms, and mixtures thereof.

7. Process according to any one of Claims 3 to 6, **characterized in that** the (poly)carbodiimide compound(s) are chosen from the compounds of formula (II) in which:
- X₁ and X₂ independently represent an oxygen atom;
- R₁ and R₂ independently represent the compound of formula (VI) below:
R₁₃-[O-CH₂-C(H)(R₁₄)]_{q}- (VI),
in which R₁₃ represents a C₁-C₄ alkyl group or a phenyl, preferably a C₁-C₄ alkyl group, more preferentially a methyl, R₁₄ represents a hydrogen atom or a C₁-C₄ alkyl group, preferably a hydrogen atom and q denotes an integer ranging from 4 to 30;
- n and z denote an integer ranging from 1 to 20, with n+z ranging from 4 to 10 and w is equal to 1;
- L₁ is a C₃-C₁₅ cycloalkylene radical such as cyclopentylene, cycloheptylene, cyclohexylene and 4,4-dicyclohexylenemethane, preferably 4,4-dicyclohexylenemethane; and
- E represents a group -O-R₃-O- in which R₃ represents a linear or branched C₁-C₁₈ alkylene radical such as methylene, propylene, butylene or ethylene, optionally interrupted with one or more heteroatoms.

8. Process according to any one of Claims 3 to 7, **characterized in that** the (poly)carbodiimide compound(s) are chosen from the compounds of formula (XII) below: 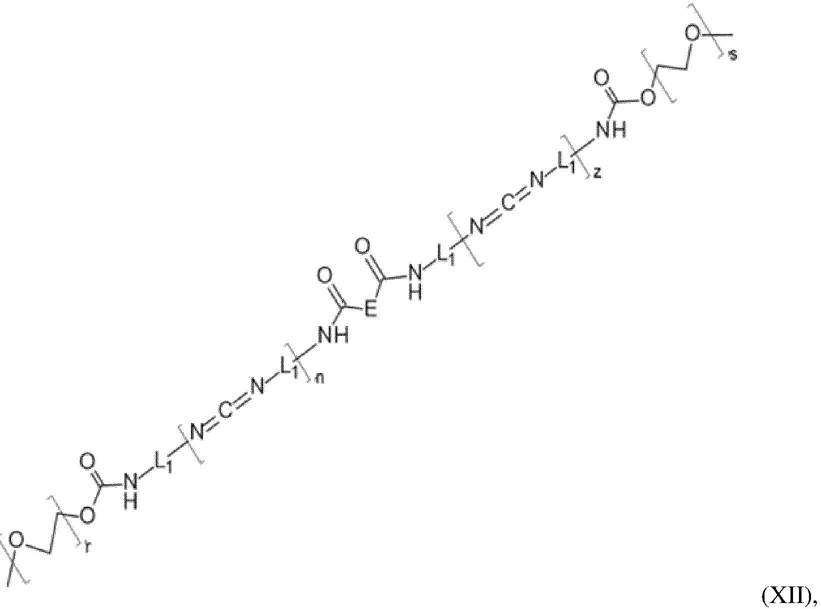 in which L₁ is 4,4-dicyclohexylenemethane, n and z denote an integer ranging from 1 to 20, with n+z ranging from 4 to 10, E represents a group -O-R₃-O- in which R₃ represents a linear or branched C₁-C₁₈ alkylene radical such as methylene, propylene, butylene or ethylene, optionally interrupted with one or more heteroatoms, and r and s denote an integer ranging from 4 to 30.

9. Process according to any one of the preceding claims, **characterized in that** the total amount of the (poly)carbodiimide compound(s) ranges from 0.01% to 20% by weight, preferably from 0.1% to 15% by weight, more preferentially from 0.2% to 10% by weight, even more preferentially from 0.5% to 8% by weight, and better still from 1% to 6% by weight, relative to the total weight of composition C.

10. Process according to any one of the preceding claims, **characterized in that** the total amount of colouring agent(s) ranges from 0.001% to 20% by weight and preferably from 0.005% to 15% by weight relative to the total weight of composition C; preferably, the colouring agent(s) are chosen from pigments.

11. Process according to any one of the preceding claims, **characterized in that** composition C also comprises at least one compound containing at least one carboxylic function.

12. Process according to Claim 11, **characterized in that** the compound(s) comprising at least one carboxylic function are chosen from silicone compounds comprising at least one carboxylic group, polyurethanes, acrylic polymers and mixtures thereof.

13. Process according to Claim 11 or 12, **characterized in that** the compound(s) containing at least one carboxylic function are in the form of aqueous dispersions of particles of polymer(s) chosen from polyurethanes, acrylic polymers and mixtures thereof, preferably in the form of aqueous dispersions of acrylic polymer particles, more preferentially in the form of aqueous dispersions of film-forming acrylic polymer particles.

14. Process according to the preceding claim, **characterized in that** the acrylic polymer(s) comprise one or more units derived from the following monomers:
a) (meth)acrylic acid; and
b) C₁ to C₃₀, more preferentially C₁ to C₂₀, better still C₁ to C₁₀, and even more particularly C₁ to C₄, alkyl (meth)acrylate.

15. Process according to any one of Claims 11 to 14, **characterized in that** the total amount of compound(s) comprising at least one carboxylic function ranges from 0.1% to 30% by weight, preferably from 0.5% to 20% by weight, more preferentially from 1% to 15% by weight and better still from 2% to 10% by weight relative to the total weight of composition C.

16. Process according to any one of Claims 1 to 8, **characterized in that** it consists in extemporaneously mixing, at the time of use, at least two compositions A and B to obtain a composition C and in spraying composition C on the keratin hair fibers, with:
- composition A comprising at least one (poly)carbodiimide compound as defined in any one of Claims 1 to 8;
- composition B comprising at least one colouring agent chosen from pigments, direct dyes and mixtures thereof.

17. Spraying system comprising:
- an airbrush;
- at least one compartment containing a composition C comprising:
a) at least one (poly)carbodiimide compound as defined in any one of Claims 1 to 9;
b) at least one colouring agent chosen from pigments, direct dyes and mixtures thereof.

18. Spraying system comprising:
- an airbrush;
- several compartments containing:
in a first compartment, a composition A comprising:
a) at least one (poly)carbodiimide compound as defined in any one of Claims 1 to 8;
in a second compartment, a composition B comprising:
b) at least one colouring agent chosen from pigments, direct dyes and mixtures thereof.

## Patentansprüche

1. Verfahren zum Färben von Keratin-Haarfasern, umfassend das Besprühen der Keratin-Haarfasern mit mindestens einer Zusammensetzung C, umfassend:
- eine oder mehrere (Poly)carbodiimidverbindungen; und
- mindestens ein Farbmittel, das aus Pigmenten, Direktfarbstoffen und Mischungen davon ausgewählt ist, unter Verwendung einer Luftspritzpistole.

2. Verfahren nach Anspruch 1, **dadurch gekennzeichnet, dass** die (Poly)carbodiimidverbindung bzw. die (Poly)carbodiimidverbindungen aus den Verbindungen der folgenden Formel (I) ausgewählt ist bzw. sind: 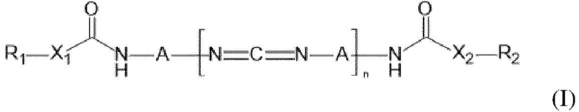 wobei:
- X₁ und X₂ unabhängig für ein Sauerstoffatom O, ein Schwefelatom S oder eine NH-Gruppe stehen;
- R₁ und R₂ unabhängig für eine Gruppe stehen, die aus einem Rest auf Kohlenwasserstoffbasis, vorzugsweise Alkyl, der gegebenenfalls durch ein oder mehrere Heteroatome unterbrochen ist, ausgewählt ist, einer Gruppe, die aus Alkoxysilyl-, Hydroxysilyl-, Acetoxysilyl-, Vinylsilyl-, Acrylalkylsilyl-, Methacrylalkylsilyl-, Crotonylalkylsilyl-, Carboxyanhydridoalkylsilyl-, Carboxyalkylsilyl-, Hydroxyalkylsilyl-, Aldehydoalkylsilyl-, Mercaptoalkylsilyl-, Norbornenylsilyl-, Acylpentadienylalkylsilyl-, Maleimidoalkylsilyl-, Sulfonylalkylsilyl-, (Meth)acrylalkyl-, Crotonylalkyl-, Alkylepoxid- wie Propylepoxid- oder Butylepoxid- und Azacyclopropangruppen ausgewählt ist, und einem Rest auf Kohlenwasserstoffbasis, vorzugsweise Alkyl, der gegebenenfalls durch ein oder mehrere Heteroatome und durch eine oder mehrere Gruppen, die aus Alkoxysilyl-, Hydroxysilyl-, Acetoxysilyl-, Vinylsilyl-, Acrylalkylsilyl-, Methacrylalkylsilyl-, Crotonylalkylsilyl-, Carboxyanhydridoalkylsilyl-, Carboxyalkylsilyl-, Hydroxyalkylsilyl-, Aldehydoalkylsilyl-, Mercaptoalkylsilyl-, Norbornenylsilyl-, Acylpentadienylalkylsilyl-, Maleimidoalkylsilyl-, Sulfonylalkylsilyl-, (Meth)acrylalkyl-, Crotonylalkyl-, Alkylepoxid- wie Propylepoxid- oder Butylepoxid- und Azacyclopropangruppen ausgewählt sind, unterbrochen ist;
- n eine ganze Zahl im Bereich von 1 bis 1000 bedeutet; und
- A für ein Monomer steht, das aus den folgenden Verbindungen ausgewählt ist:

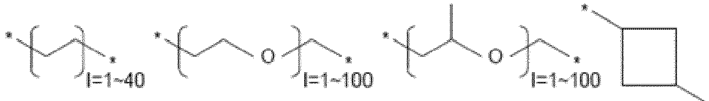

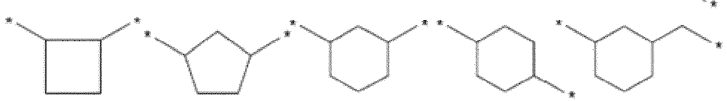

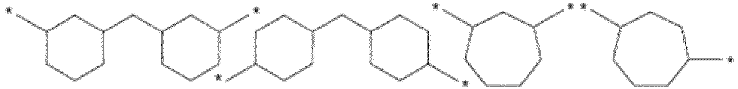

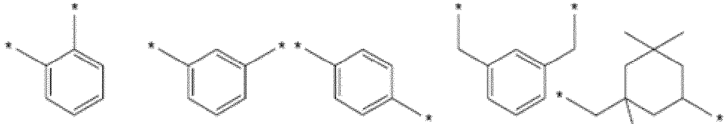

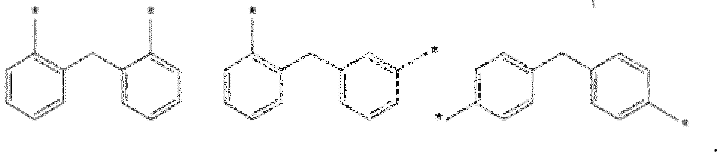

3. Verfahren nach Anspruch 1, **dadurch gekennzeichnet, dass** die (Poly)carbodiimidverbindung bzw. die (Poly)carbodiimidverbindungen aus den Verbindungen der folgenden Formel (II) ausgewählt ist bzw. sind: 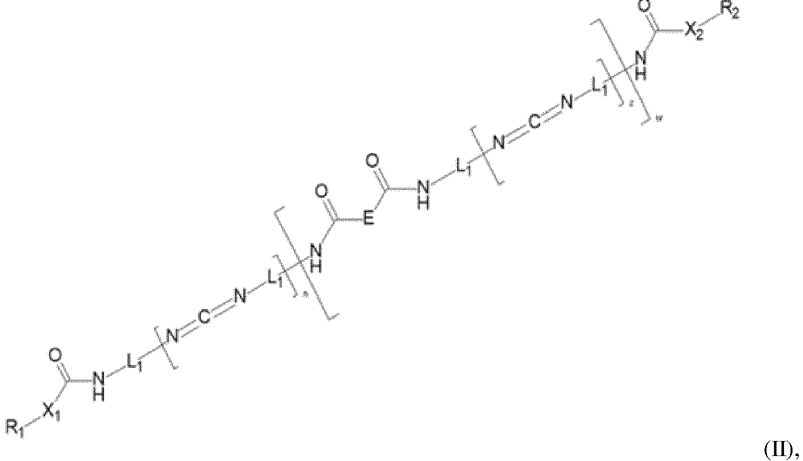 wobei:
- X₁ und X₂ unabhängig für ein Sauerstoffatom O, ein Schwefelatom S oder eine NH-Gruppe stehen;
- R₁ und R₂ unabhängig für einen Rest auf Kohlenwasserstoffbasis, der gegebenenfalls durch ein oder mehrere Heteroatome unterbrochen ist, stehen;
- n und z eine ganze Zahl im Bereich von 1 bis 20 mit n+z ≥ 2 bedeuten und w eine ganze Zahl im Bereich von 1 bis 3 bedeutet;
- L₁ unabhängig für einen zweiwertigen aliphatischen C₁-C₁₈-Rest auf Kohlenwasserstoffbasis, einen C₃-C₁₅-Cycloalkylenrest, eine C₃-C₁₂-Heterocycloalkylengruppe oder eine C₆-C₁₄-Arylengruppe und Mischungen davon steht;
- E unabhängig für eine Gruppe steht, die ausgewählt ist aus:
-O-R₃-O-; -S-R₄-S-; -R₅-N(R₆)-R₄-N(R₆)-R₅-;
wobei R₃ und R₄ unabhängig für einen zweiwertigen Rest auf Kohlenwasserstoffbasis, der gegebenenfalls durch ein oder mehrere Heteroatome unterbrochen ist, stehen;
- R₅ unabhängig für eine kovalente Bindung oder einen gesättigten zweiwertigen Rest auf Kohlenwasserstoffbasis, der gegebenenfalls durch ein oder mehrere Heteroatome unterbrochen ist, steht;
- R₆ unabhängig für ein Wasserstoffatom oder einen Rest auf Kohlenwasserstoffbasis, der gegebenenfalls durch ein oder mehrere Heteroatome unterbrochen ist, steht.

4. Verfahren nach Anspruch 3, **dadurch gekennzeichnet, dass** die (Poly)carbodiimidverbindung bzw. die (Poly)carbodiimidverbindungen aus den Verbindungen der Formel (II) ausgewählt ist bzw. sind, wobei:
- X₁ und X₂ unabhängig für ein Sauerstoffatom stehen;
- R₁ und R₂ unabhängig aus Dialkylaminoalkoholen, Hydroxycarbonsäurealkylestern und Monoalkylethern von (Poly)alkylenglykol, in denen eine Hydroxylgruppe entfernt wurde, und Mischungen davon ausgewählt sind;
- n und z eine ganze Zahl im Bereich von 1 bis 20 mit n+z ≥ 2 bedeuten und w gleich 1 ist;
- L1\ aus einem zweiwertigen aliphatischen C₁-C₁₈-Rest auf Kohlenwasserstoffbasis, einem C₃-C₁₅-Cycloalkylenrest, einer C₃-C₁₂-Heterocycloalkylengruppe oder einer C₆-C₁₄-Arylengruppe und Mischungen davon ausgewählt ist;
- E unabhängig für eine Gruppe steht, die ausgewählt ist aus:
-O-R₃-O-; -S-R₄-S-; -R₅-N(R₆)-R₄-N(R₆)-R₅-;
wobei R3 und R4 unabhängig aus einem C₆-C₁₄-Arylenrest, einem C₃-C₁₂-Cycloalkylenrest, einem linearen oder verzweigten C₁-C₁₈-Alkylenrest, der gegebenenfalls durch ein oder mehrere Heteroatome unterbrochen ist, und Mischungen davon ausgewählt sind;
- dann, wenn R₅ keine kovalente Bindung ist, R₅ aus einem C₆-C₁₄-Arylenrest, einem C₃-C₁₂-Cycloalkylenrest, einem linearen oder verzweigten C₁-C₁₈-Alkylenrest, der gegebenenfalls durch ein oder mehrere Heteroatome unterbrochen ist, und Mischungen davon ausgewählt ist; und
- R₆ aus einem C₆-C₁₄-Arylenrest, einem C₃-C₁₂-Cycloalkylenrest, einem linearen oder verzweigten C₁-C₁₈-Alkylenrest, der gegebenenfalls durch ein oder mehrere Heteroatome unterbrochen ist, und Mischungen davon ausgewählt ist.

5. Verfahren nach Anspruch 3 oder 4, **dadurch gekennzeichnet, dass** die (Poly)carbodiimidverbindung bzw. die (Poly)carbodiimidverbindungen aus den Verbindungen der Formel (II) ausgewählt ist bzw. sind, wobei:
- X₁ und X₂ unabhängig für ein Sauerstoffatom stehen;
- R₁ und R₂ unabhängig für Monoalkylether von (Poly)alkylenglykol, in denen eine Hydroxylgruppe entfernt wurde, stehen;
- n und z eine ganze Zahl im Bereich von 1 bis 20 mit n+z ≥ 2 bedeuten und w gleich 1 ist;
- L₁ für einen C₃-C₁₅-Cycloalkylenrest steht;
- E unabhängig für eine Gruppe steht, die ausgewählt ist aus:
-O-R₃-O-; -S-R₄-S-; -R₅-N(R₆)-R₄-N(R₆)-R₅-;
wobei R₃ und R₄ unabhängig aus einem C₆-C₁₄-Arylenrest, einem C₃-C₁₂-Cycloalkylenrest, einem linearen oder verzweigten C₁-C₁₈-Alkylenrest, der gegebenenfalls durch ein oder mehrere Heteroatome unterbrochen ist, und Mischungen davon ausgewählt sind;
- dann, wenn R₅ keine kovalente Bindung ist, R₅ aus einem C₆-C₁₄-Arylenrest, einem C₃-C₁₂-Cycloalkylenrest, einem linearen oder verzweigten C₁-C₁₈-Alkylenrest, der gegebenenfalls durch ein oder mehrere Heteroatome unterbrochen ist, und Mischungen davon ausgewählt ist; und
- R₆ aus einem C₆-C₁₄-Arylenrest, einem C₃-C₁₂-Cycloalkylenrest, einem linearen oder verzweigten C₁-C₁₈-Alkylenrest, der gegebenenfalls durch ein oder mehrere Heteroatome unterbrochen ist, und Mischungen davon ausgewählt ist.

6. Verfahren nach einem der Ansprüche 3 bis 5, **dadurch gekennzeichnet, dass** die (Poly)carbodiimidverbindung bzw. die (Poly)carbodiimidverbindungen aus den Verbindungen der Formel (II) ausgewählt ist bzw. sind, wobei:
- X₁ und X₂ unabhängig für ein Sauerstoffatom stehen;
- R₁ und R₂ unabhängig für die Verbindung der folgenden Formel (VI) stehen:
R₁₃-[O-CH₂-C(H)(R₁₄)]_{q}- (VI),
wobei R₁₃ für eine C₁-C₄-Alkylgruppe oder ein Phenyl, vorzugsweise eine C₁-C₄-Alkylgruppe, weiter bevorzugt ein Methyl, steht, R₁₄ für ein Wasserstoffatom oder eine C₁-C₄-Alkylgruppe, vorzugsweise ein Wasserstoffatom, steht und q eine ganze Zahl im Bereich von 4 bis 30 bedeutet;
- n und z eine ganze Zahl im Bereich von 1 bis 20 mit n+z im Bereich von 4 bis 10 bedeuten und w gleich 1 ist;
- L₁ für einen C₃-C₁₅-Cycloalkylenrest wie Cyclopentylen, Cycloheptylen, Cyclohexylen und 4,4-Dicyclohexylenmethan steht; und
- E für eine Gruppe -O-R₃-O- steht, wobei R₃ aus einem C₆-C₁₄-Arylenrest, einem C₃-C₁₂-Cycloalkylenrest, einem linearen oder verzweigten C₁-C₁₈-Alkylenrest, der gegebenenfalls durch ein oder mehrere Heteroatome unterbrochen ist, und Mischungen davon ausgewählt ist.

7. Verfahren nach einem der Ansprüche 3 bis 6, **dadurch gekennzeichnet, dass** die (Poly)carbodiimidverbindung bzw. die (Poly)carbodiimidverbindungen aus den Verbindungen der Formel (II) ausgewählt ist bzw. sind, wobei:
- X₁ und X₂ unabhängig für ein Sauerstoffatom stehen;
- R₁ und R₂ unabhängig für die Verbindung der folgenden Formel (VI) stehen:
R₁₃-[O-CH₂-C(H)(R₁₄)]_{q}- (VI),
wobei R₁₃ für eine C₁-C₄-Alkylgruppe oder ein Phenyl, vorzugsweise eine C₁-C₄-Alkylgruppe, weiter bevorzugt ein Methyl, steht, R₁₄ für ein Wasserstoffatom oder eine C₁-C₄-Alkylgruppe, vorzugsweise ein Wasserstoffatom, steht und q eine ganze Zahl im Bereich von 4 bis 30 bedeutet;
- n und z eine ganze Zahl im Bereich von 1 bis 20 mit n+z im Bereich von 4 bis 10 bedeuten und w gleich 1 ist;
- L₁ für einen C₃-C₁₅-Cycloalkylenrest wie Cyclopentylen, Cycloheptylen, Cyclohexylen und 4,4-Dicyclohexylenmethan, vorzugsweise 4,4-Dicyclohexylenmethan, steht; und
- E für eine Gruppe -O-R₃-O- steht, wobei R₃ für einen linearen oder verzweigten C₁-C₁₈-Alkylenrest wie Methylen, Propylen, Butylen oder Ethylen, der gegebenenfalls durch ein oder mehrere Heteroatome unterbrochen ist, steht.

8. Verfahren nach einem der Ansprüche 3 bis 7, **dadurch gekennzeichnet, dass** die (Poly)carbodiimidverbindung bzw. die (Poly)carbodiimidverbindungen aus den Verbindungen der folgenden Formel (XII) ausgewählt ist bzw. sind: 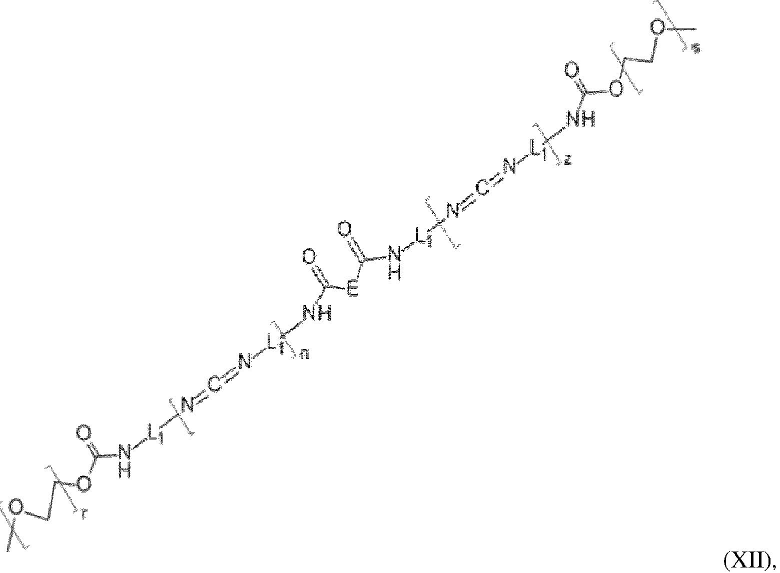 wobei L₁ für 4,4-Dicyclohexylenmethan steht, n und z eine ganze Zahl im Bereich von 1 bis 20 mit n+z im Bereich von 4 bis 10 bedeuten, E für eine Gruppe -O-R₃-O- steht, wobei R₃ für einen linearen oder verzweigten C₁-C₁₈-Alkylenrest wie Methylen, Propylen, Butylen oder Ethylen, der gegebenenfalls durch ein oder mehrere Heteroatome unterbrochen ist, steht und r und s eine ganze Zahl im Bereich von 4 bis 30 bedeuten.

9. Verfahren nach einem der vorhergehenden Ansprüche, **dadurch gekennzeichnet, dass** die Gesamtmenge der (Poly)carbodiimidverbindung bzw. der (Poly)carbodiimidverbindungen im Bereich von 0,01 bis 20 Gew.-%, vorzugsweise von 0,1 bis 15 Gew.-%, weiter bevorzugt von 0,2 bis 10 Gew.-%, noch weiter bevorzugt von 0,5 bis 8 Gew.-% und noch besser von 1 bis 6 Gew.-%, bezogen auf das Gesamtgewicht der Zusammensetzung C, liegt.

10. Verfahren nach einem der vorhergehenden Ansprüche, **dadurch gekennzeichnet, dass** die Gesamtmenge an Farbmittel bzw. Farbmitteln im Bereich von 0,001 bis 20 Gew.-% und vorzugsweise von 0,005 bis 15 Gew.-%, bezogen auf das Gesamtgewicht der Zusammensetzung C, liegt; wobei das Farbmittel bzw. die Farbmittel vorzugsweise aus Pigmenten ausgewählt ist bzw. sind.

11. Verfahren nach einem der vorhergehenden Ansprüche, **dadurch gekennzeichnet, dass** die Zusammensetzung C außerdem mindestens eine Verbindung mit mindestens einer Carboxylfunktion umfasst.

12. Verfahren nach Anspruch 11, **dadurch gekennzeichnet, dass** die Verbindung bzw. Verbindungen mit mindestens einer Carboxylfunktion aus Silikonverbindungen mit mindestens einer Carboxylgruppe, Polyurethanen, Acrylpolymeren und Mischungen davon ausgewählt ist bzw. sind.

13. Verfahren nach Anspruch 11 oder 12, **dadurch gekennzeichnet, dass** die Verbindung bzw. Verbindungen mindestens mindestens einer Carboxylfunktion in Form von wässrigen Dispersionen von Teilchen von Polymer bzw. Polymeren, das bzw. die aus Polyurethanen, Acrylpolymeren und Mischungen davon ausgewählt ist bzw. sind, vorzugsweise in Form von wässrigen Dispersionen von Acrylpolymerteilchen, weiter bevorzugt in Form von wässrigen Dispersionen von Teilchen von filmbildenden Acrylpolymeren, vorliegt bzw. vorliegen.

14. Verfahren nach dem vorhergehenden Anspruch, **dadurch gekennzeichnet, dass** das Acrylpolymer bzw. die Acrylpolymere eine oder mehrere Einheiten umfasst bzw. umfassen, die sich von den folgenden Monomeren ableiten:
a) (Meth)acrylsäure und
b) C₁- bis C₃₀-, weiter bevorzugt C₁- bis C₂₀-, noch besser C₁- bis C₁₀- und noch spezieller C₁- bis C₄-Alkyl(meth)acrylat.

15. Verfahren nach einem der Ansprüche 11 bis 14, **dadurch gekennzeichnet, dass** die Gesamtmenge der Silikonverbindung bzw. Silikonverbindungen mit mindestens einer Carboxylfunktion im Bereich von 0,1 bis 30 Gew.-%, vorzugsweise von 0,5 bis 20 Gew.-%, weiter bevorzugt von 1 bis 15 Gew.-% und noch besser von 2 bis 10 Gew.-%, bezogen auf das Gesamtgewicht der Zusammensetzung C, liegt.

16. Verfahren nach einem der Ansprüche 1 bis 8, **dadurch gekennzeichnet, dass** es aus dem unmittelbaren Mischen von mindestens zwei Zusammensetzungen A und B zum Zeitpunkt der Anwendung unter Erhalt einer Zusammensetzung C und aus dem Aufsprühen der Zusammensetzung C auf die Keratin-Haarfasern besteht, wobei:
- Zusammensetzung A mindestens eine (Poly)carbodiimidverbindung gemäß einem der Ansprüche 1 bis 8 umfasst;
- Zusammensetzung B mindestens ein Farbmittel, das aus Pigmenten, Direktfarbstoffen und Mischungen davon ausgewählt ist, umfasst.

17. Sprühsystem, umfassend:
- eine Luftspritzpistole;
- mindestens ein Kompartiment, das eine Zusammensetzung C umfasst, die Folgendes umfasst:
a) mindestens eine (Poly)carbodiimidverbindung gemäß einem der Ansprüche 1 bis 9;
b) mindestens ein Farbmittel, das aus Pigmenten, Direktfarbstoffen und Mischungen davon ausgewählt ist.

18. Sprühsystem, umfassend:
- eine Luftspritzpistole;
- mehrere Kompartimente, enthaltend:
in einem ersten Kompartiment eine Zusammensetzung A, umfassend:
a) mindestens eine (Poly)carbodiimidverbindung gemäß einem der Ansprüche 1 bis 8;
in einem zweiten Kompartiment eine Zusammensetzung B, umfassend:
b) mindestens ein Farbmittel, das aus Pigmenten, Direktfarbstoffen und Mischungen davon ausgewählt ist.

## Revendications

1. Procédé de coloration des cheveux comprenant la pulvérisation sur les cheveux d'au moins une composition C comprenant :
- un ou plusieurs composés (poly)carbodiimide ; et
- au moins un agent colorant choisi parmi les pigments, les colorants directs, et leurs mélanges,
à l'aide d'un aérographe.

2. Procédé selon la revendication 1, **caractérisée en ce que** le ou les composé(s) (poly)carbodiimide est (sont) choisi(s) parmi les composés de formule (I) suivante : 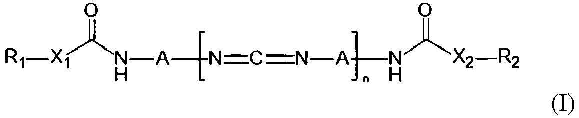 dans laquelle :
- X₁ et X₂ représentent, indépendamment, un atome d'oxygène O, un atome de soufre S ou un groupement NH ;
- R₁ et R₂ représentent indépendamment un groupement choisi parmi un radical hydrocarboné, de préférence alkyle, éventuellement interrompu par un ou plusieurs hétéroatome(s), un groupement choisi parmi les groupements alkoxysilyle, hydroxysilyle, acétoxysilyle, vinylsilyle, acrylalkylsilyle, méthacrylalkylsilyle, crotonylalkylsilyle, carboxyanhydridoalkylsilyle, carboxyalkylsilyle, hydroxyalkylsilyle, aldéhydoalkylsilyle, mercaptoalkylsilyle, norbornénylsilyle, acylpentadiénylalkylsilyle, maléimidoalkylsilyle, sulphonylalkylsilyle, (méth)acrylalkyle, crotonylalkyle, alkylépoxyde tel que propylépoxyde ou butylépoxyde, et azacyclopropane, et un radical hydrocarboné, de préférence alkyle, éventuellement interrompu par un ou plusieurs hétéroatome(s) et par un ou plusieurs groupements choisis parmi les groupements alkoxysilyle, hydroxysilyle, acétoxysilyle, vinylsilyle, acrylalkylsilyle, méthacrylalkylsilyle, crotonylalkylsilyle, carboxyanhydridoalkylsilyle, carboxyalkylsilyle, hydroxyalkylsilyle, aldéhydoalkylsilyle, mercaptoalkylsilyle, norbornénylsilyle, acylpentadiénylalkylsilyle, maléimidoalkylsilyle, sulphonylalkylsilyle, (méth)acrylalkyle, crotonylalkyle, alkylépoxyde tels que propylépoxyde ou butylépoxyde, et azacyclopropane ;
- n désigne un nombre entier allant de 1 à 1000 ; et
- A est un monomère choisi parmi les composés ci-dessous :

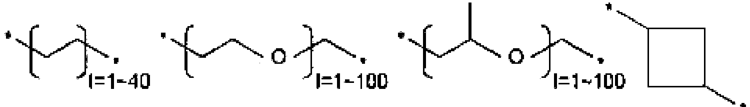

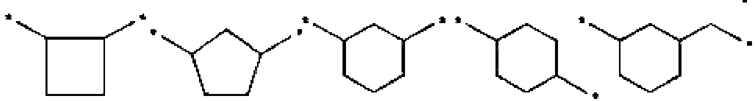

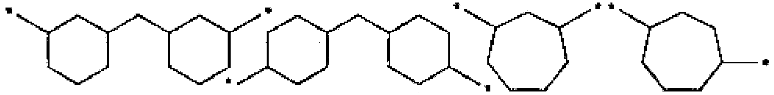

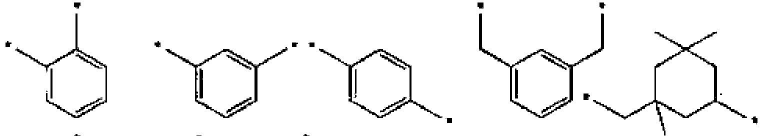

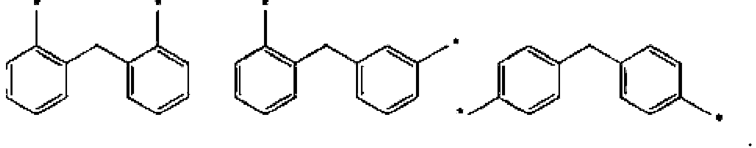

3. Procédé selon la revendication 1, **caractérisé en ce que** le ou les composé(s) (poly)carbodiimide est (sont) choisi(s) parmi les composés de formule (II) suivante : 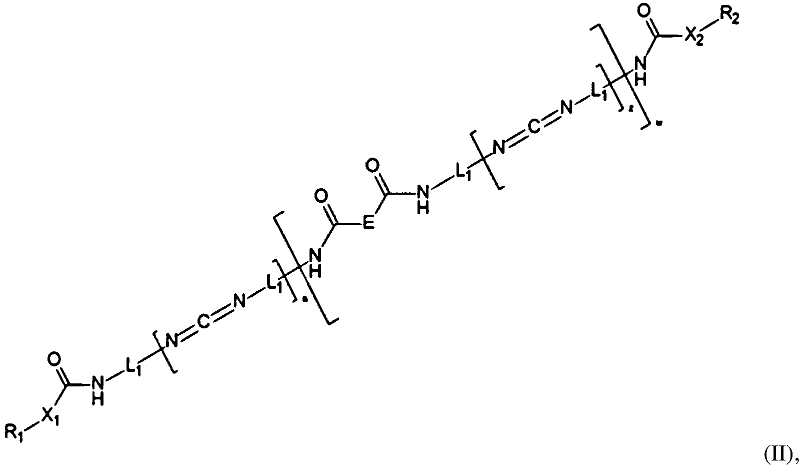 dans laquelle
- X₁ et X₂ représentent, indépendamment, un atome d'oxygène O, un atome de soufre S ou un groupement NH ;
- R₁ et R₂ représentent indépendamment un radical hydrocarboné éventuellement interrompu par un ou plusieurs hétéroatome(s) ;
- n et z désignent un nombre entier allant de 1 à 20, avec n+z ≥ 2 et w désigne un nombre entier allant de 1 à 3 ;
- L₁ représente indépendamment un radical hydrocarboné aliphatique divalent en C₁-C₁₈, un radical cycloalkylène en C₃-C₁₅, un groupement hétérocycloalkylène en C₃-C₁₂, ou un groupement arylène en C₆-C₁₄, et leurs mélanges ;
- E représente indépendamment un groupement choisi parmi :
-O-R₃-O-; -S-R₄-S-; -R₅-N(R₆)-R₄-N(R₆)-R₅-,
dans lequel R₃ et R₄ représentent indépendamment un radical hydrocarboné divalent éventuellement interrompu par un ou plusieurs hétéroatome(s) ;
- R₅ représente indépendamment une liaison covalente ou un radical hydrocarboné saturé divalent, éventuellement interrompu par un ou plusieurs hétéroatome(s) ;
- R₆ représente indépendamment un atome d'hydrogène, ou un radical hydrocarboné éventuellement interrompu par un ou plusieurs hétéroatome(s).

4. Procédé selon la revendication 3, **caractérisé en ce que** le ou les composé(s) (poly)carbodiimide est (sont) choisi(s) parmi les composés de formule (II) dans laquelle :
- X₁ et X₂ représentent indépendamment un atome d'oxygène ;
- R₁ et R₂, sont choisis indépendamment parmi les dialkylaminoalcools, les esters alkyliques d'acide hydroxycarboxylique et les éthers monoalkyliques de (poly)alkylèneglycol, dans lesquels un groupement hydroxy a été retiré, et leurs mélanges ;
- n et z désignent un nombre entier allant de 1 à 20, avec n+z ≥ 2 et w est égal à 1 ;
- L1 est choisi parmi un radical hydrocarboné aliphatique divalent en C₁-C₁₈, un radical cycloalkylène en C₃-C₁₅, un groupement hétérocycloalkylène en C₃-C₁₂, ou un groupement arylène en C₆-C₁₄, et leurs mélanges ;
- E représente indépendamment un groupement choisi parmi :
-
-O-R₃-O-; -S-R₄-S-; -R₅-N(R₆)-R₄-N(R₆)-R₅-;
dans lequel R3 et R4 sont choisis indépendamment parmi un radical arylène en C₆-C₁₄, un radical cycloalkylène en C₃-C₁₂, un radical alkylène linéaire ou ramifié en C₁-C₁₈, éventuellement interrompu par un ou plusieurs hétéroatome(s), et leurs mélanges ;
- lorsque R₅ n'est pas une liaison covalente, R₅ est choisi parmi un radical arylène en C₆-C₁₄, un radical cycloalkylène en C₃-C₁₂, un radical alkylène linéaire ou ramifié en C₁-C₁₈, éventuellement interrompu par un ou plusieurs hétéroatome(s), et leurs mélanges ; et
- R₆ est choisi parmi un radical arylène en C₆-C₁₄, un radical cycloalkylène en C₃-C₁₂, un radical alkylène linéaire ou ramifié en C₁-C₁₈, éventuellement interrompu par un ou plusieurs hétéroatome(s), et leurs mélanges.

5. Procédé selon la revendication 3 ou 4, **caractérisé en ce que** le ou les composé(s) (poly)carbodiimide est (sont) choisi(s) parmi les composés de formule (II) dans laquelle :
- X₁ et X₂ représentent indépendamment un atome d'oxygène ;
- R₁ et R₂, sont indépendamment des éthers monoalkyliques de (poly)alkylèneglycol, dans lesquels un groupement hydroxy a été retiré ;
- n et z désignent un nombre entier allant de 1 à 20, avec n+z ≥ 2 et w est égal à 1 ;
- L₁ est un radical cycloalkylène en C₃-C₁₅ ;
- E représente indépendamment un groupement choisi parmi :
-O-R₃-O-; -S-R₄-S-; -R₅-N(R₆)-R₄-N(R₆)-R₅- ;
dans lequel R₃ et R₄ sont choisis indépendamment parmi un radical arylène en C₆-C₁₄, un radical cycloalkylène en C₃-C₁₂, un radical alkylène linéaire ou ramifié en C₁-C₁₈, éventuellement interrompu par un ou plusieurs hétéroatome(s), et leurs mélanges ;
- lorsque R₅ n'est pas une liaison covalente, R₅ est choisi parmi un radical arylène en C₆-C₁₄, un radical cycloalkylène en C₃-C₁₂, un radical alkylène linéaire ou ramifié en C₁-C₁₈, éventuellement interrompu par un ou plusieurs hétéroatome(s), et leurs mélanges ; et
- R₆ est choisi parmi un radical arylène en C₆-C₁₄, un radical cycloalkylène en C₃-C₁₂, un radical alkylène linéaire ou ramifié en C₁-C₁₈, éventuellement interrompu par un ou plusieurs hétéroatome(s), et leurs mélanges.

6. Procédé selon l'une quelconque des revendications 3 à 5, **caractérisé en ce que** le ou les composé(s) (poly)carbodiimide est (sont) choisi(s) parmi les composés de formule (II) dans laquelle :
- X₁ et X₂ représentent indépendamment un atome d'oxygène ;
- R₁ et R₂, représentent indépendamment le composé de formule (VI) suivante :
R₁₃-[O-CH₂-C(H)(R₁₄)]_{q}- (VI),
dans laquelle R₁₃ représente un groupe alkyle en C₁-C₄ ou un phényle, de préférence un groupe alkyle en C₁-C₄, plus préférentiellement un méthyle, R₁₄ représente un atome d'hydrogène ou un groupe alkyl en C₁-C₄, de préférence un atome d'hydrogène et q désigne un nombre entier allant de 4 à 30 ;
- n et z désignent un nombre entier allant de 1 à 20, avec n+z allant de 4 à 10 et w est égal à 1 ;
- L₁ est un radical cycloalkylène en C₃-C₁₅ tel que cyclopentylène, cycloheptylène, cyclohexylène et le 4,4-dicyclohexylène méthane, et
- E représente un groupement -O-R₃-O- dans lequel R₃ est choisi parmi un radical arylène en C₆-C₁₄, un radical cycloalkylène en C₃-C₁₂, un radical alkylène linéaire ou ramifié en C₁-C₁₈, éventuellement interrompu par un ou plusieurs hétéroatome(s), et leurs mélanges.

7. Procédé selon l'une quelconque des revendications 3 à 6, **caractérisé en ce que** le ou les composé(s) (poly)carbodiimide est (sont) choisi(s) parmi les composés de formule (II) dans laquelle :
- X₁ et X₂ représentent indépendamment un atome d'oxygène ;
- R₁ et R₂, représentent indépendamment le composé de formule (VI) suivante :
R₁₃-[O-CH₂-C(H)(R₁₄)]_{q}- (VI),
dans laquelle R₁₃ représente un groupe alkyle en C₁-C₄ ou un phényle, de préférence un groupe alkyle en C₁-C₄, plus préférentiellement un méthyle, R₁₄ représente un atome d'hydrogène ou un groupe alkyle en C₁-C₄, de préférence un atome d'hydrogène et q désigne un nombre entier allant de 4 à 30 ;
- n et z désignent un nombre entier allant de 1 à 20, avec n+z allant de 4 à 10, et w est égal à 1 ;
- L₁ est un radical cycloalkylène en C₃-C₁₅ tel que le cyclopentylène, le cycloheptylène, le cyclohexylène et le 4,4-dicyclohexylène méthane, de préférence le 4,4-dicyclohexylène méthane ; et
- E représente un groupement- -O-R₃-O- dans lequel R₃ représente un radical alkylène linéaire ou ramifié en C₁-C₁₈ tel que le méthylène, propylène, butylène, éthylène, éventuellement interrompu par un ou plusieurs hétéroatome(s).

8. Procédé selon l'une quelconque des revendications 3 à 7, **caractérisé en ce que** le ou les composé(s) (poly)carbodiimide est(sont) choisi(s) parmi les composés de formule (XII) suivante : 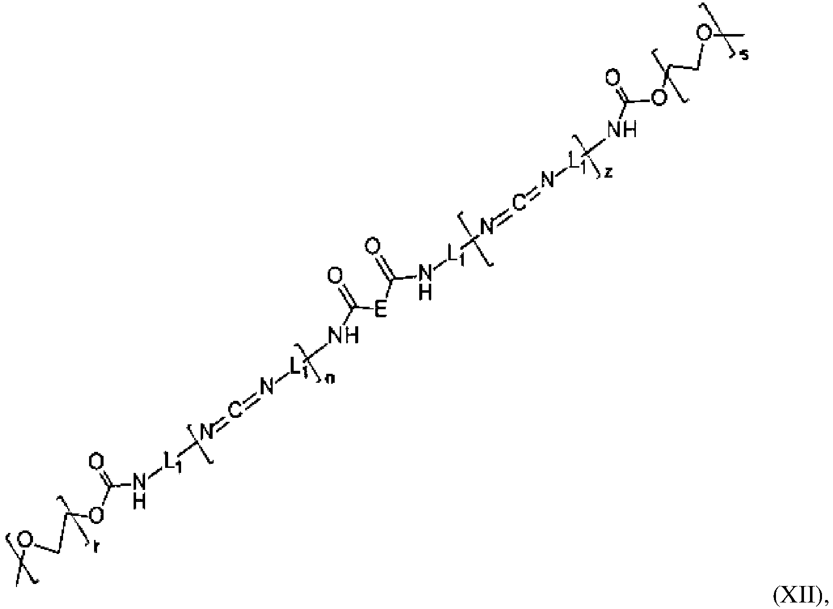 dans laquelle L₁ est le 4,4-dicyclohexylène méthane, n et z désignent un nombre entier allant de 1 à 20, avec n+z allant de 4 à 10, E représente un groupement- -O-R₃-O- dans lequel R₃ représente un radical alkylène linéaire ou ramifié en C₁-C₁₈ tel que méthylène, propylène, butylène éthylène, éventuellement interrompu par un ou plusieurs hétéroatome(s), et r et s désignent un nombre entier allant de 4 à 30.

9. Procédé selon l'une quelconque des revendications précédentes, **caractérisé en ce que** la quantité totale du ou des composé(s) (poly)carbodiimide va de 0,01 à 20% en poids, de préférence de 0,1 à 15% en poids, plus préférentiellement de 0,2 à 10% en poids, encore plus préférentiellement de 0,5 à 8% en poids, mieux de 1 à 6% en poids par rapport au poids total de la composition C.

10. Procédé selon l'une quelconque des revendications précédentes, **caractérisé en ce que** la quantité totale en agent(s) colorant(s) va de 0,001 à 20 % en poids, de préférence de 0,005 à 15 % en poids par rapport au poids total de la composition C, de préférence le ou les agents colorants sont choisis parmi les pigments.

11. Procédé selon l'une quelconque des revendications précédentes, **caractérisé en ce que** la composition C comprend en outre au moins un composé ayant au moins une fonction carboxylique.

12. Procédé selon la revendication 11, **caractérisé en ce que** le ou les composés comprenant au moins une fonction carboxylique sont choisis parmi les composés siliconés comprenant au moins un groupement carboxylique, les polyuréthanes, les polymères acryliques et leurs mélanges.

13. Procédé selon la revendication 11 ou 12, **caractérisé en ce que** le ou les composés ayant au moins une fonction carboxylique sont sous forme de dispersions aqueuses de particules de polymère(s) choisis parmi les polyuréthanes, les polymères acryliques et leurs mélanges, de préférence sous forme de dispersions aqueuses de particules de polymères acryliques, plus préférentiellement sous forme de dispersions aqueuses de particules de polymères acryliques filmogènes.

14. Procédé selon la revendication précédente, **caractérisé en ce que** le ou les polymères acryliques comprennent un ou plusieurs motifs issus des monomères suivants :
a) acide (méth)acrylique ; et
b) (méth)acrylate d'alkyle en C₁ à C₃₀, plus préférentiellement en C₁ à C₂₀, encore mieux en C₁ à C₁₀, et encore plus particulièrement en C₁ à C₄.

15. Procédé selon l'une quelconque des revendications 11 à 14, **caractérisé en ce que** la quantité totale en composé(s) comprenant au moins une fonction carboxylique va de 0,1 à 30% en poids, de préférence de 0,5 à 20% en poids, plus préférentiellement de 1 à 15% en poids, mieux de 2 à 10% en poids par rapport au poids total de la composition C.

16. Procédé selon l'une quelconque des revendications 1 à 8, **caractérisé en ce qu'**il consiste à mélanger extemporanément au moment de l'emploi au moins deux compositions A et B pour obtenir une composition C et à pulvériser la composition C sur les cheveux, avec :
- la composition A comprenant au moins un composé (poly)carbodiimide tel que défini à l'une quelconque des revendications 1 à 8 ;
- la composition B comprenant au moins un agent colorant choisi parmi les pigments, les colorants directs, et leurs mélanges

17. Système de pulvérisation comprenant :
- un aérographe ;
- au moins un compartiment contenant une composition C comprenant :
a) au moins un composé (poly)carbodiimide tel que défini à l'une quelconque des revendications 1 à 9 ;
b) au moins un agent colorant choisi parmi les pigments, les colorants directs, et leurs mélanges.

18. Système de pulvérisation comprenant :
- un aérographe ;
- plusieurs compartiments contenant :
dans un premier compartiment, une composition A comprenant :
a) au moins un composé (poly)carbodiimide tel que défini à l'une quelconque des revendications 1 à 8 ;
dans un deuxième compartiment, une composition B comprenant :
b) au moins un agent colorant choisi parmi les pigments, les colorants directs, et leurs mélanges.
